# EUROPÄISCHE PATENTSCHRIFT

(11) **EP 0 492 501 B1**
(45) Veröffentlichungstag und Bekanntmachung des Hinweises auf die Patenterteilung: **20.11.1996**
(21) Anmeldenummer: 91121937.6
(22) Anmeldetag: 20.12.1991
(51) Int. Cl.: C07D 251/70, B01F 17/32, C07D 403/12, C08G 73/06

(54) **Grenzflächenaktive Triazinverbindungen, Verfahren zu ihrer Herstellung und ihre Verwendung**
Surface-active triazine compounds, process for their preparation and their use
Dérivés tensioactifs de triazine, procédé pour leur préparation et leur utilisation

(30) Priorität: 21.12.1990 DE 4041215
(43) Veröffentlichungstag der Anmeldung: 01.07.1992
(73) Patentinhaber: HOECHST AKTIENGESELLSCHAFT, 65926 Frankfurt am Main (DE)
(72) Erfinder: Az, Rainer, Dr., W-6238 Hofheim am Taunus (DE); Schwab, Wolfgang, Dr., W-6092 Kelsterbach (DE); Schnaitmann, Dieter, Dr., W-6239 Eppstein/Taunus (DE); Dietz, Erwin, Dr., W-6233 Kelkheim (Taunus) (DE)

(56) Entgegenhaltungen:
- US-A- 2 394 306
- US-A- 4 347 352

## Beschreibung

Die vorliegende Erfindung liegt auf dem technischen Gebiet der oberflächenaktiven Verbindungen.

Bei den derzeit in der Technik hauptsächlich angewendeten Triazinverbindungen handelt es sich um Pflanzenschutzmittel, Melaminharze und vor allem um Textilfarbstoffe. Aufgebaut sind solche Verbindungen in der Regel aus einem Triazinring, manchmal auch zwei Triazinringen, welche durch Chromophore substituiert sind. Im allgemeinen werden diese Farbstoffe durch Substituenten an den Chromophoren wasserlöslich gemacht. Zur Beeinflussung der färberischen Eigenschaften kann der Triazinring noch weitere Substituenten, beispielsweise Aminogruppen mit aliphatischen Resten, Vinylsulfongruppen und Alkoxygruppen, tragen. Solche Verbindungen werden zur Einfärbung textiler Materialien verwendet. Es sind auch oligomere und polymere Triazinverbindungen, die aus mehr als zwei Triazinringen aufgebaut sind, bekannt. In der US-A-4 314 001 und US-A-4 347 352 sind polymere Triazinverbindungen der allgemeinen Formel I (siehe Formelschema), wobei Y und Z Aminogruppen enthaltende Substituenten darstellen, beschrieben, welche sich als Beize für wasserlösliche Farbstoffe auf Cellulose, als Fixiermittel für Pigmente auf Cellulose oder als oberflächenaktive Agenzien für wäßrige Pigmentdispersionen eignen. In der US-PS 3 622 339 sind ebenfalls polymere Triazinverbindungen der allgemeinen Formel II (siehe Formelschema) genannt, wobei R¹ ein Amino-, Hydroxyalkyl- oder Mercapto-Gruppen enthaltender Rest ist, R², R³, R⁴ und R⁵ jeweils Wasserstoff oder eine Alkylgruppe und Y¹ und Y² jeweils eine diradikalische, gegebenenfalls substituierte Alkylen- oder Arylen-Gruppe darstellen, welche als Antischleiermittel in Filmemulsionen dienen. In der US-PS 4 228 281 werden polymere Triazinringe enthaltende Dicarbonsäuren der allgemeinen Formel III (siehe Formelschema) beschrieben, worin R¹, R², R³ und R⁵ jeweils Wasserstoff oder einen C₁-C₂₂-Alkyl-Kohlenwasserstoffrest, R⁴ einen diradikalischen C₂-C₁₈-Kohlenwasserstoffrest und R⁶ einen diradikalischen C₁-C₁₂-Kohlenwasserstoffrest bedeuten, welche zur Synthese von Polyestern mit einem Glasumwandlungspunkt über 100°C verwendet werden.

Der vorliegenden Erfindung liegt die Aufgabe zugrunde, polymere wasserlösliche Triazinverbindungen zur Verfügung zu steilen, die farblos oder nur schwach gefärbt sind, gute grenzflächenaktive Eigenschaften aufweisen und gegebenenfalls auch zur Pigmentierung oder zur Beschichtung der Oberfläche von Pigmenten geeignet sind. Die Aufgabe wird durch eine neue Klasse von basische und saure Gruppen enthaltenden Triazinverbindungen der allgemeinen Formel IV (siehe Formelschema) gelöst, wobei X eine Gruppe der Formel IVa (siehe Formelschema) darstellt, in der A¹ ein Brückenglied NR³ ist, worin R³ Wasserstoff oder einen C₁-C₂₂-Alkylrest oder einen C₃-C₂₂-Alkenylrest, vorzugsweise einen C₁-C₄-Alkylrest oder Wasserstoff, insbesondere Methyl oder Wasserstoff, darstellt, der gegebenenfalls durch eine OH-Gruppe substituiert ist, D¹ eine Arylengruppe oder eine verzweigte oder unverzweigte C₂-C₁₂-Alkylengruppe ist, welche gegebenenfalls durch ein oder mehrere Brückenglieder aus der Gruppe A² unterbrochen ist, und A² unabhängig von A¹ dieselbe Gruppe von Substituenten wie A¹ bedeutet, und R¹ und R² unabhängig voneinander verzweigte oder unverzweigte C₁-C₂₀-Alkylgruppen oder C₃-C₂₀-Alkenylgruppen, vorzugsweise C₁-C₆-Alkylgruppen, sind, oder in der R¹ und R² zusammen mit dem Stickstoffatom einen ungesättigten oder gesättigten fünf- oder sechsgliedrigen Ring bilden, welcher gegebenenfalls zusätzlich ein oder zwei Atome Stickstoff, Sauerstoff und/oder Schwefel als weitere Heteroatome im Ring enthält, oder wobei X eine Gruppe der Formel IVb (siehe Formelschema) darstellt, worin R⁴ C₁-C₆-Alkyl bedeutet, sowie Y¹ und Y² unabhängig voneinander jeweils eine Gruppe der Formel IVc (siehe Formelschema) darstellen, in der A³ unabhängig von A¹ ist und ein Brückenglied aus derselben Gruppe von Substituenten wie A¹ bedeutet, D² unabhängig von D¹ ist und dieselbe Gruppe von Substituenten wie D¹, CH₂ oder eine direkte Bindung bedeutet, und E¹ COOM, SO₃M, OSO₃M oder OPO₃M₂ ist, worin M Wasserstoff, Metall, insbesondere Alkalimetall oder die stöchiometrische Menge eines Erdalkalimetalls oder ein Ammoniumion ist, welches gegebenenfalls durch aliphatische, aromatische und araliphatische Reste substituiert ist, und Z eine Gruppe der Formel IVd (siehe Formelschema) darstellt, in der A⁴ unabhängig von A¹ ist und ein Brückenglied aus derselben Gruppe von Substituenten wie A¹ bedeutet und Z* die Gruppe CO, verzweigtes oder unverzweigtes C₂-C₂₅-Alkylen, C₅-C₆-Cycloalkylen oder C₆-C₁₄-Arylen, vorzugsweise C₂-C₆-Alkylen oder Phenylen, ist, welches gegebenenfalls durch COOM, SO₃M, Cl, OH oder Alkoxygruppen substituiert ist, wobei M die obengenannten Bedeutungen hat und wobei in den cyclischen Verbindungen gegebenenfalls die Kohlenstoffatome teilweise durch die Heteroatome Stickstoff, Sauerstoff und/oder Schwefel ersetzt sind, oder wobei Z ein Brückenglied der allgemeinen Formeln IVe und IVf (siehe Formelschema) darstellt, in denen A⁵ eine Gruppe der Formeln CR⁵R⁶, NR⁷, O, SO, SO₂ und CO, bevorzugt CR⁵R⁶, NR⁷ oder O, ist, wobei R⁵ und R⁶ unabhängig voneinander verzweigte oder unverzweigte C₁-C₄-Alkylgruppen sowie Wasserstoff sein können und wobei R⁷ unabhängig von R³ dieselbe Gruppe von Substituenten wie R³ bedeutet, oder Z eine beliebige Kombination der Gruppen der Formeln IVd, IVe und/oder IVf ist, und m eine ganze Zahl von 1 bis 100 bedeutet.

Bevorzugt sind Verbindungen der allgemeinen Formel IV (siehe Formelschema), wobei X eine Gruppe der Formel IVa (siehe Formelschema) darstellt, in der A¹ ein Brückenglied NR³ ist, worin R³ Wasserstoff oder einen C₁-C₂₂-Alkylrest oder einen C₃-C₂₂-Alkenylrest, vorzugsweise einen C₁-C₄-Alkylrest oder Wasserstoff, insbesondere Methyl oder Wasserstoff, darstellt, der gegebenenfalls durch eine OH-Gruppe substituiert ist, D¹ eine Arylengruppe oder eine verzweigte oder unverzweigte C₂-C₁₂-Alkylengruppe ist, welche gegebenenfalls durch ein oder mehrere Brückenglieder aus der Gruppe A² unterbrochen ist, und A² unabhängig von A¹ dieselbe Gruppe von Substituenten wie A¹ bedeutet, und R¹ und R² unabhängig voneinander verzweigte oder unverzweigte C₁-C₂₀-Alkylgruppen oder C₃-C₂₀-Alkenylgruppen, vorzugsweise C₁-C₆-Alkylgruppen, sind, oder in der R¹ und R² zusammen mit dem Stickstoffatom einen ungesättigten oder gesättigten fünf- oder sechsgliedrigen Ring bilden, welcher gegebenenfalls zusätzlich ein oder zwei Atome Stickstoff oder Sauerstoff als weitere Heteroatome im Ring enthält, oder wobei X eine Gruppe der Formel IVb (siehe Formelschema) darstellt, worin R⁴ C₁-C₆-Alkyl bedeutet, sowie Y¹ und Y² unabhängig voneinander jeweils eine Gruppe der Formel IVc (siehe Formelschema) darstellen, in der A³ unabhängig von A¹ ist und ein Brückenglied aus derselben Gruppe von Substituenten wie A¹ bedeutet, D² unabhängig von D¹ ist und dieselbe Gruppe von Substituenten wie D¹, CH₂ oder eine direkte Bindung bedeutet, und E¹ COOM oder SO₃M ist, worin M Wasserstoff, Alkalimetall oder die stöchiometrische Menge eines Erdalkalimetalls, oder ein Ammoniumion, welches gegebenenfalls durch aliphatische, aromatische und araliphatische Reste substituiert ist, und Z eine Gruppe der Formel IVd (siehe Formelschema) darstellt, in der A⁴ unabhängig von A¹ ist und ein Brückenglied aus derselben Gruppe von Substituenten wie A¹ bedeutet, und Z* die Gruppe CO, verzweigtes oder unverzweigtes C₂-C₂₅-Alkylen, C₅-C₆-Cycloalkylen oder Phenylen, vorzugsweise C₂-C₆-Alkylen oder Phenylen, ist, welches gegebenenfalls durch COOM oder SO₃M substituiert ist, wobei M die obengenannten Bedeutungen hat, und wobei in den cyclischen Verbindungen gegebenenfalls die Kohlenstoffatome teilweise durch die Heteroatome Stickstoff und/oder Sauerstoff ersetzt sind, oder Z eine Kombination der Gruppen der Formel IVd darstellt, und m eine ganze Zahl von 1 bis 100 bedeutet.

Besonders bevorzugt sind Verbindungen der allgemeinen Formel IV (siehe Formelschema), wobei X eine Gruppe der Formel IVg (siehe Formelschema) darstellt, in der R⁸ Methyl oder Wasserstoff ist, und D¹ eine verzweigte oder unverzweigte C₂-C₆-Alkylengruppe oder eine Phenylengruppe ist, und R¹ und R² unabhängig voneinander verzweigte oder unverzweigte C₁-C₆-Alkylgruppen sind, oder in der R¹ und R² zusammen mit dem Stickstoffatom einen ungesättigten oder gesättigten fünf- oder sechsgliedrigen Ring bilden, welcher gegebenenfalls zusätzlich ein oder zwei Atome Stickstoff und/oder Sauerstoff als weitere Heteroatome im Ring enthält, sowie Y¹ und Y² unabhängig voneinander jeweils eine Gruppe der Formel IVh (siehe Formelschema) darstellen, in der R⁹ unabhängig von R⁸ ist und ein Substituent aus derselben Gruppe wie R⁸ bedeutet, und D² unabhängig von D¹ ist und dieselbe Gruppe von Substituenten wie D¹, CH₂ oder eine direkte Bindung bedeutet, und E¹ COOM oder SO₃M ist, worin M Wasserstoff, Alkalimetall oder die stöchiometrische Menge eines Erdalkalimetalls, oder ein Ammoniumion, welches gegebenenfalls durch aliphatische, aromatische und araliphatische Reste substituiert ist, und Z eine Gruppe der Formel IVi (siehe Formelschema) darstellt, in der R¹⁰ Methyl oder Wasserstoff ist, und Z* die Gruppe C₂-C₆-Alkylen oder Phenylen ist, welches gegebenenfalls durch COOM oder SO₃M substituiert ist, wobei M die obengenannten Bedeutungen hat, oder wobei Z eine Gruppe der Formel IVj (siehe Formelschema) darstellt, in der Z** die Gruppe N-C₁-C₂₀-Alkyl, NH oder O bedeutet, und m eine ganze Zahl von 1 bis 30 bedeutet.

Die erfindungsgemäßen Verbindungen sind farblos oder nur schwach gefärbt, was sie zu einer breiten Anwendung im Bereich der grenzflächenaktiven Substanzen befähigt, da sie den Farbton des Anwendungsmediums nicht beeinflussen.

Nach dem Stand der Technik erfolgt die Synthese von Triazinverbindungen durch nukleophile Substitution der Halogenatome an Cyanurhalogeniden, insbesondere Cyanurchlorid, mit Amin-, Hydroxy- oder Mercaptoderivaten bei Temperaturen von -10 bis +200°C (J. Am. Chem. Soc. Bd. 73 (1951), 2981-2996). Dabei läßt sich die Substitutionsreaktion durch Temperaturführung und pH-Wert-Kontrolle, die mit säurebindenden Mitteln, wie zum Beispiel Natronlauge oder Soda, durchgeführt wird, selektiv steuern. Die Substitution aller drei Halogenatome mit unterschiedlichen Resten läßt sich dabei in einem einzigen Reaktionsgefäß sukzessive durchführen. Dieses Verfahren ist aber auf die erfindungsgemäßen Verbindungen nicht anwendbar, da dadurch Y¹ und Y² nur statistisch verteilt einführbar sind. Ist die Reaktivität von Y¹-H und Y²-H stark voneinander verschieden, ist gegebenenfalls nur noch Y¹ beziehungsweise Y² substituierbar.
Die Produkte der allgemeinen Formel IV mit Y¹ = Y², die bei der Synthese in einem einzigen Reaktionsgefäß entstehen, besitzen physikalische und chemische Eigenschaften, die für den anwendungstechnischen Zweck nur wenig geeignet sind.

Die vorliegende Erfindung betrifft weiterhin ein Verfahren zur Synthese einer Verbindung der allgemeinen Formel IV, das dadurch gekennzeichnet ist, daß man in getrennten Behältern zunächst eine Verbindung der Formel Y¹-H (erster Reaktionsansatz) und Y²-H (zweiter Reaktionsansatz) jeweils in wäßriger Lösung mit Cyanurhalogenid, vorzugsweise Cyanurchlorid, bei einer Temperatur von 0 bis 10°C, vorzugsweise 0 bis 5°C, umsetzt, anschließend das im ersten Reaktionsansatz entstandene Zwischenprodukt mit einer Verbindung der Formel X-H und das im zweiten Reaktionsansatz entstandene Zwischenprodukt mit einer Verbindung der Formel ZH₂ jeweils bei einer Temperatur von 20 bis 60°C, vorzugsweise 35 bis 45°C, umsetzt, danach die beiden Reaktionsansätze zusammengibt und mit 0,1 bis 0,6 Mol ZH₂ pro Mol eingesetztem Cyanurhalogenid bei einer Temperatur von 70 bis 150°C, vorzugsweise 75 bis 95°C, umsetzt.

Durch diese Reaktionsführung wird erreicht, daß die beiden endständigen Triazinringe jeweils die saure Gruppe Y¹ sowie den Substituenten X mit einer basischen Funktion, insbesondere einem tertiären Stickstoffatom enthalten, während alle übrigen Triazinringe mit der sauren Gruppe Y² substituiert sind. Die Synthese wird in zwei getrennten Reaktionsgefäßen durchgeführt, wobei die Reaktion bevorzugt in Wasser als Lösungsmittel durchgeführt wird, dem geringe Mengen eines Tensids, beispielsweise ein sulfoniertes Fettsäurederivat, zugegeben ist.

Im ersten Schritt werden die Gruppen Y¹ beziehungsweise Y² in separaten Rührgefäßen (A) und (B) in Wasser an das Cyanurhalogenid gebunden. Dazu werden pro Mol Cyanurhalogenid 0,8 bis 1,2 Mol Y¹-H beziehungsweise Y²-H verwendet und die Reaktion bei O bis 10°C, vorzugsweise O bis 5°C, durchgeführt. In idealisierter Form laufen die Reaktionen nach Reaktionsschema 1 ab (siehe Formelschema).

Im zweiten Schritt wird zum einen die Gruppe X in Reaktionsgefäß (A) (Reaktionsprodukt V), zum anderen die Gruppe Z in Reaktionsgefäß (B) (Reaktionsprodukt VI) bei 20 bis 60°C, bevorzugt 35 bis 45°C, substituiert (Reaktionsschema 2, siehe Formelschema).
In diesem Schritt werden jeweils 0,8 bis 1,2 Mol X-H beziehungsweise 0,3 bis 1,2 Mol H-Z-H pro Mol Cyanurhalogenid verwendet. Anschließend werden bei 70 bis 150°C, bevorzugt 75 bis 95°C, die beiden erhaltenen Zwischenprodukte VII und VIII über Z verknüpft, wobei 0,1 bis 0,6 Mol H-Z-H pro Mol Cyanurhalogenid verwendet werden (Reaktionsschema 3, siehe Formelschema). Diese Menge an H-Z-H kann zweckmäßigerweise auch schon bei der Umsetzung von VI zu VIII zugegen sein.

Zur Neutralisation der bei der Reaktion entstehenden Säure HHal wird eine solche Menge eines säurebindenden Mittels, beispielsweise Natron- oder Kalilauge, Soda, Natriumhydrogencarbonat oder Pottasche, in jeder Reaktionsstufe sukzessive mit fortschreitender Reaktion (Abnahme des pH-Wertes) oder auf einmal zugegeben, daß der pH-Wert der Reaktionsgemische bei 6 bis 9 liegt. Die auf diese Weise erhaltene Reaktionsmischung läßt sich in den erfindungsgemäßen Anwendungen direkt einsetzen, gegebenenfalls nach Abfiltration etwa vorhandener fester Bestandteile. Weiterhin läßt sich das Reaktionsprodukt durch Zugabe von Säuren, beispielsweise Salzsäure oder Schwefelsäure, aus der Lösung ausfällen und durch Filtration abtrennen. Das Lösungsmittel läßt sich aus der Reaktionsmischung durch Vakuum, erhöhte Temperatur oder Sprühtrocknung entfernen und entstandenes Salz durch Zugabe von organischen Lösungsmitteln, bevorzugt Dimethylsulfoxid, entfernen. Die Salzabtrennung kann auch durch Membrantrennverfahren erfolgen.
Als grenzflächenaktives Mittel kann das Produkt nach der Säurefällung in Form des noch feuchten Preßkuchens, in getrockneter Form sowie auch in Form einer Amin-, Alkali- oder Erdalkalisalzlösung verwendet werden. Amine zur Salzbildung sind beispielsweise: Methylamin, Mono-, Di- und Triethylamin, Mono-, Di- und Triethanolamin, Dimethylethanolamin, Methyldiethanolamin und Dimethylaminomethylpropanol.

Beispiele für die Verbindungen der Formel X-H sind N,N-Dimethyl-p- phenylendiamin, N,N-Diethyl-p-phenylendiamin, Aminobenzyldimethylamin, 2-Dimethylaminoethylamin, 3-Diethylaminopropylamin, 3-Dimethylaminopropylamin, N¹,N¹-Diethyl-1,4-pentandiamin, N,N-Dimethylneopentandiamin, 2-Pyrrolidinoethylamin oder N-(3-Aminopropyl)-imidazol.

Beispiele für die Verbindungen der Formeln Y¹-H und Y²-H sind o-, m- und p-Aminobenzoesäure, o-, m- und p-Aminobenzolsulfonsäure, Glycin, Alanin, β-Alanin, 4-Aminobuttersäure, Amidosulfonsäure oder 2-Aminoethansulfonsäure.

Beispiele für die Verbindungen der Formeln ZH₂ sind 1,2-Diaminoethan, 1,2-Diaminopropan, 1,3-Diaminopropan, 1,4-Diaminobutan, 1,6-Diaminohexan, 1,8-Diaminooctan, 1,12-Diaminododecan, Neopentandiamin, 4,4'-Diaminodicyclohexylmethan, 4,4'-Diaminodiphenylmethan, N-(2-Aminoethyl)-1,2-ethandiamin, N-(2-Aminopropyl)-1,3-propandiamin, 4,4'-Diaminodiphenylether oder 1,4-Phenylendiamin-2-sulfonsäure.

Die erfindungsgemäßen Verbindungen mit der allgemeinen Formel IV liegen je nach pH-Wert in der protonierten Form, als Zwitterion oder in der deprotonierten Form vor.

Die erfindungsgemäßen Verbindungen sind im allgemeinen in polaren sowie in protischen Lösungsmitteln, gegebenenfalls bei erhöhtem pH-Wert, gut löslich. Die grenzflächenaktiven Eigenschaften der erfindungsgemäßen Verbindungen zeigen sich zum Beispiel in der Verringerung der Oberflächenspannung einer wäßrigen Lösung. Diese läßt sich nach der Ringabreißmethode (DIN 53914) bestimmen und ergibt je nach Verbindung bei einer Meßtemperatur von 25°C in wäßriger aminalkalischer Lösung (pH 9,0) Werte von 35 bis 45 mN/m (Vergleichswert einer wäßrigen aminalkalischen Lösung bei pH 9,0 : 70 mN/m).

Die erfindungsgemäßen Verbindungen eignen sich als Dispergier-, Emulgier- und Verteilungsmittel von Feststoffen in wäßrigen Medien. Die vorliegende Erfindung betrifft deshalb auch die Verwendung der erfindungsgemäßen Verbindungen als grenzflächenaktive Mittel, als Dispergier-, Emulgier- oder Verteilungsmittel von Feststoffen in wäßrigen Medien. Insbesondere können die erfindungsgemäßen Verbindungen in Dispersionen verwendet werden, die als Feststoffe beispielsweise Mineralien, Pflanzenschutz- und Schädlingsbekämpfungsmittel, Farbstoffe, Pigmente und optische Aufheller enthalten. Des weiteren eignen sich die erfindungsgemäßen Verbindungen als Färberei- und Stabilisierungsmittel, zur Massefärbung von Regeneratcellulose sowie als Dispergierhilfsmittel für den wäßrigen Flexodruck. Besonders hervorzuheben ist die Eignung der erfindungsgemäßen Verbindungen für die Herstellung hochpigmentierter, gut fließfähiger Pigmentpräparationen anorganischer und organischer Pigmente, wie beispielsweise Azopigmente, Chinacridone, Flavanthron-, Anthanthron- und Pyranthronkörper, Derivate der Naphthalintetracarbonsäure und der Perylentetracarbonsäure, des Thioindigos, des Dioxazins, der Isoindoline, Isoindolinone und der Diketopyrrolopyrrole, verlackte Pigmente, wie die Magnesium-, Calcium-, Strontium-, Barium-, Aluminium-, Mangan- und Nickellacke von säuregruppenhaltigen Farbstoffen, von Phthalocyaninpigmenten sowie von entsprechenden Pigmentmischungen. Unter den vorgenannten Pigmentpräparationen werden einerseits Pigmentdispersionen in konzentrierter Form, die durch Verdünnen mit Bindemitteln auf Basis wäßriger Polyacrylat-, Polyester- und Polyurethansysteme in die endgültige Lackformulierung überführt werden können, andererseits Pigmentzubereitungen, die pulverförmig in das Anwendungsmedium eingearbeitet werden, verstanden.

Gegenstand der Erfindung sind auch Pigmentdispersionen, gekennzeichnet im wesentlichen durch den Gehalt an mindestens einer der Verbindungen der Formel IV und mindestens einem anorganischen und/oder organischen, vorzugsweise polycyclischen, Pigment.
Gegenstand der Erfindung sind weiterhin Pigmentzubereitungen organischer, vorzugsweise polycyclischer, Pigmente, insbesondere von Perylen- und Dioxazinpigmenten sowie von Chinacridonen. Die erfindungsgemäßen Verbindungen der Formel IV können einzeln oder als Gemisch sowie auch in Kombination mit anderen nichtionogenen, anionenaktiven oder auch kationenaktiven Tensiden oder Gemischen davon eingesetzt werden. Weiterhin können sie gemeinsam mit Gerüstsubstanzen oder anderen üblichen Zusätzen oder Hilfsstoffen zur Anwendung kommen. Die Zusammensetzung der genannten Pigmentzubereitungen kann in weiten Grenzen schwanken.

Die bevorzugten Pigmentzubereitungen bestehen im wesentlichen aus
a) 99,5 bis 50 Gew.-%, vorzugsweise 97 bis 70 Gew.-%, mindestens eines organischen, vorzugsweise polycyclischen Pigments,
b) 0,5 bis 30 Gew.-%, vorzugsweise 3 bis 20 Gew.-%, mindestens einer Verbindung der Formel IV und
c) 0 bis 20 Gew.-%, vorzugsweise 0 bis 10 Gew.-%, an weiteren üblichen Zusatzstoffen, beispielsweise Schaum- und Viskositätsregulatoren, Tenside, Antiabsetzmittel, Netzmittel und Konservierungsmittel.

Gegenstand der vorliegenden Erfindung ist ferner ein Verfahren zur Herstellung der Pigmentzubereitungen, dadurch gekennzeichnet, daß die Oberfläche der Pigmente mit mindestens einer der Verbindungen der Formel IV beschichtet wird. Je nach Ausführungsform kann das Aufbringen dieser Verbindungen auf die Pigmentteilchen entweder in wäßriger Suspension, in organischen Lösemitteln oder in Gemischen von Wasser und organischen Lösemitteln erfolgen. Es gibt eine Reihe von Möglichkeiten, die erfindungsgemäßen Verbindungen auf die Pigmentoberfläche aufzubringen. Es kann dies während oder nach der Pigmentsynthese, bei oder nach einem Finishprozeß erfolgen. Die Zugabe der Verbindungen der Formel IV kann auch bei der Einarbeitung der Pigmente ins Anwendungsmedium erfolgen. Beim Aufbringen der erfindungsgemäßen Verbindungen auf die Pigmentoberfläche in wäßriger Suspension oder in Wasser-Lösemittelgemischen wird das Pigment anschließend üblicherweise durch Filtration isoliert. In der Regel hat dabei der pH-Wert einen Einfluß auf die Pigmenteigenschaften. Bevorzugt ist ein pH-Wert von 7 bis 12, besonders bevorzugt von 7,5 bis 9.

Gegenstand der Erfindung ist auch die Verwendung der erfindungsgemäßen Pigmentdispersionen und Pigmentzubereitungen zum Pigmentieren und Färben natürlicher und synthetischer Materialien. Bevorzugt ist die Verwendung bei der Herstellung wäßriger Lack-, Dispersions- und Druckfarben. Besonders bevorzugt ist die Verwendung der Pigmentzubereitungen sowie der Pigmentdispersionen zum Pigmentieren von wäßrigen Lacksystemen. Die erfindungsgemäßen Pigmentzubereitungen und Pigmentdispersionen liefern in allen gängigen wäßrigen Bindemittelsystemen wie Polyacrylaten, Polyurethanen und Polyestern reine brilliante Farbtöne bei hoher Farbstärke und hoher Transparenz. Die Pigmentpasten zeigen gute rheologische Eigenschaften bei hohen Pigmentgehalten und pH-Konstanz, auch nach mehrwöchiger Warmlagerung. Zur Eignungsprüfung werden die in den nachfolgenden Beispielen beschriebenen Verbindungen entweder auf die Pigmente aufgebracht, oder sie werden zur Dispergierung von Pigmenten in wäßrigen Lacksystemen eingesetzt. Als Lacksysteme werden wäßrige Polyacrylat-, Polyester- und Polyurethansysteme gewählt. An den mit diesen Lacken erstellten Einbrennlackierungen werden die anwendungstechnischen Eigenschaften (Farbstärke, Farbton, Transparenz) geprüft. Zur Beurteilung der coloristischen Kriterien werden die verdünnten Volltonlacke entweder mit einem Handcoater (Spiralrakel, 24 µm Naßfilmstärke) auf transparente Polyesterfolie oder mit einem Filmziehgerät (Spiral-Filmziehgerät, 15 µm Naßfilmstärke) auf Kontrastkarton aufgezogen. Die Beurteilung der Rheologie erfolgte visuell anhand der Stammpasten nach der Dispergierung.

Dazu wurde die nachstehende fünfstufige Skala zugrundegelegt:
5 dünnflüssig
4 flüssig
3 dickflüssig
2 leicht gestockt
1 gestockt

Nach dem Verdünnen des Mahlgutes auf die Pigmentendkonzentration kann die Viskosität (Visk) mit einem Visko-Spatel nach Rossmann, Typ 301 der Firma Erichsen, Iserlohn, bestimmt werden. Vor der Lackierung wurde die gewünschte Viskosität so eingestellt, daß sie einer bestimmten Auslaufzeit im Fordbecher (Düse 4 mm Durchmesser), angegeben in Sekunden (s), entsprach.

Farbstärke und Farbton wurden anhand von Weißabmischungen mit TiO₂ oder von Metallic-Abmischungen mit Aluminium bewertet.
Die coloristische Beurteilung erfolgte anhand der nachfolgend aufgeführten 6-stufigen Bewertungsskala:
1 wenig
2 etwas
3 merklich
4 deutlich
5 wesentlich
6 bedeutend

Die Glanzmessungen erfolgten unter einem Winkel von 20° nach DIN 67530 (ASTMD 523) mit einem "multigloss"-Glanzmeßgerät der Fa. Byk-Mallinckrodt, Wesel. Die in den Beispielen angegebenen Werte für den Glanz sind dimensionslose Reflexionswerte. In den nachstehenden Herstellungs- und Anwendungsbeispielen beziehen sich Teile auf Gewichtsteile, Prozentangaben sind Gewichtsprozente. Die verwendeten Abkürzungen haben folgende Bedeutung:
"VT 3 % P" bedeutet "Vollton 3 % Pigmentgehalt".
"1 : 10 TiO₂" bedeutet "Mischung aus 1 Teil Buntpigment und 10 Teilen Titandioxid-Weißpigment".
"50 : 50 Met" bedeutet "Mischung aus 50 Teilen Buntpigment und 50 Teilen Aluminiumpigment".
"VE-Wasser" bedeutet "vollentsalztes Wasser".

### Synthesebeispiele

1) Zur Synthese der Verbindung der Formel IV mit
   X = -NH-CH₂-CH₂-CH₂-N(C₂H₅)₂, Y¹ = Y² = -NH-C₆H₄-SO₃H,
   Z = -NH-CH₂-CH₂-NH- und m = 2 wurden im Rührgefäß (A) zu 400 Teilen einer Wasser-Eis-Mischung, die 0,2 Teile eines sulfonierten Fettsäurederivates und 18,5 Teile Cyanurchlorid enthielt, bei 0 bis 5°C 17,3 Teile Sulfanilsäure gegeben und durch Zugabe von 0,2 normaler NaOH-Lösung der pH-Wert auf 6 bis 9 gehalten. Nach der Laugenzugabe wurde etwa eine Stunde bei 0 bis 5°C nachgerührt. Anschließend wurden bei etwa 40°C 13,0 Teile Diethylaminopropylamin zugegeben und durch Zugabe von 0,2 normaler NaOH-Lösung der pH-Wert auf 6 bis 9 gehalten. Nach der Laugenzugabe wurde etwa eine Stunde bei etwa 40°C nachgerührt. Im Rührgefäß (B) wurden zu 400 Teilen einer Wasser-Eis-Mischung, die 0,2 Teile eines sulfonierten Fettsäurederivates und 18,5 Teile Cyanurchlorid enthielt, bei 0 bis 5°C 17,3 Teile Sulfanilsäure gegeben und durch Zugabe von 0,2 normaler NaOH-Lösung der pH-Wert auf 6 bis 9 gehalten. Nach der Laugenzugabe wurde etwa eine Stunde bei 0 bis 5°C nachgerührt. Anschließend wurden bei ca. 40°C 3,0 Teile Diaminoethan zugegeben und durch Zugabe von 0,2 normaler NaOH-Lösung der pH-Wert auf 6 bis 9 gehalten. Nach der Laugenzugabe wurde etwa eine Stunde bei 40°C nachgerührt. Es wurde nun der Inhalt des Rührgefäßes (B) in das Rührgefäß (A) gegeben und bei 80°C 6,0 Teile Diaminoethan zugegeben und durch Zugabe von 0,2 normaler NaOH-Lösung der pH-Wert auf 6 bis 9 gehalten. Nach der Laugenzugabe wurde bei 95°C noch etwa fünf Stunden nachgerührt. Nachdem die Reaktionsmischung auf Raumtemperatur abgekühlt war, wurde mit konzentrierter Salzsäure ein pH-Wert von 1,5 eingestellt, der entstandene Niederschlag abfiltriert und getrocknet.
   IR (KBr, cm⁻¹) 3300, 3100, 1630, 1590, 1550, 1500, 1220, 1170, 1030, 1000
2) Zur Synthese der Verbindung der Formel IV mit
   X = -NH-CH₂-CH₂-CH₂-imidazolyl, Y¹ = -NH-CH₂-CH₂-SO₃H,
   Y² = -NH-CH₂-CH₂-COOH, Z = -NH-C₆H₄-NH- und m = 2 wurden in Rührgefäß (A) zu 400 Teilen einer Wasser-Eis-Mischung, die 0,4 Teile eines sulfonierten Fettsäurederivates und 36,9 Teile Cyanurchlorid enthielt, bei 0 bis 5°C 25,0 Teile Taurin gegeben und durch Zugabe von 0,2 normaler NaOH-Lösung der pH-Wert auf 6 bis 9 gehalten. Nach der Laugenzugabe wurde etwa eine Stunde bei 0 bis 5°C nachgerührt. Anschließend wurden bei ca. 40°C 25,0 Teile N-(3-Aminopropyl)-imidazol, gelöst in 50 Teilen Wasser, zugegeben und durch Zugabe von 0,2 normaler NaOH-Lösung der pH-Wert auf 6 bis 9 gehalten. Nach der Laugenzugabe wurde etwa eine Stunde nachgerührt. Im Rührgefäß (B) wurden zu 400 Teilen einer Wasser-Eis-Mischung, die 0,4 Teile eines sulfonierten Fettsäurederivates und 36,9 Teile Cyanurchlorid enthielt, bei 0 bis 5°C 17,8 Teile β-Alanin, gelöst in 100 Teilen Wasser, gegeben und durch Zugabe von 0,2 normaler NaOH-Lösung der pH-Wert auf 6 bis 9 gehalten. Nach der Laugenzugabe wurde etwa eine Stunde bei 0 bis 5°C nachgerührt. Anschließend wurden bei ca. 40°C 32,4 Teile p-Phenylendiamin zugegeben und durch Zugabe von 0,2 normaler NaOH-Lösung der pH-Wert auf 6 bis 9 gehalten. Nach der Laugenzugabe wurde etwa eine Stunde bei ca. 40°C nachgerührt.
   Es wurde nun der Inhalt des Rührgefäßes (B) in das Rührgefäß (A) gegeben und bei ca. 80°C der pH-Wert durch Zugabe von 0,2 normaler NaOH-Lösung auf 6 bis 9 gehalten. Nach der Laugenzugabe wurde bei ca. 95°C noch etwa fünf Stunden nachgerührt. Nachdem die Reaktionsmischung auf Raumtemperatur abgekühlt war, wurde mit konzentrierter Salzsäure ein pH-Wert von 1,5 eingestellt, der entstandene Niederschlag abfiltriert und getrocknet.
   IR (KBr, cm⁻¹) 3300, 3100, 1630, 1570, 1500, 1410, 1220, 1160, 1030

### Beispiele 3 - 8

Die nachfolgenden Verbindungen der Formel IV mit den Substituenten X, Y¹, Y² und Z wurden entsprechend den Beispielen 1 und 2 umgesetzt:

| Nr. | X | Y¹, Y² jeweils | Z | m |
|---|---|---|---|---|
| 3 | -NH-(CH₂)₃-N(C₂H₅)₂ | -NH-C₆H₄-SO₃H | -NH-C₆H₄-NH- | 2 |
| 4 | -NH-(CH₂)₃-N(C₂H₅)₂ | -NH-C₆H₄-SO₃H | -NH-C₆H₄-NH- | 5 |
| 5 | -NH-(CH₂)₃-CH(CH₃)N(C₂H₅)₂ | -NH-CH₂-CH₂-SO₃H | -NH-C₆H₄-NH- | 5 |
| 6 | -NH-(CH₂)₃-N(C₂H₅)₂ | -NH-CH₂-CH₂-SO₃H | -NH-C₆H₄-NH- | 2 |
| 7 | -NH-(CH₂)₃-N(C₂H₅)₂ | -NH-CH₂-CH₂-SO₃H | -NH-C₆H₃(SO₃H)-NH- | 2 |
| 8 | -NH-(CH₂)₃-imidazolyl | -NH-C₆H₄-COOH | -NH-C₆H₄-NH- | 2 |

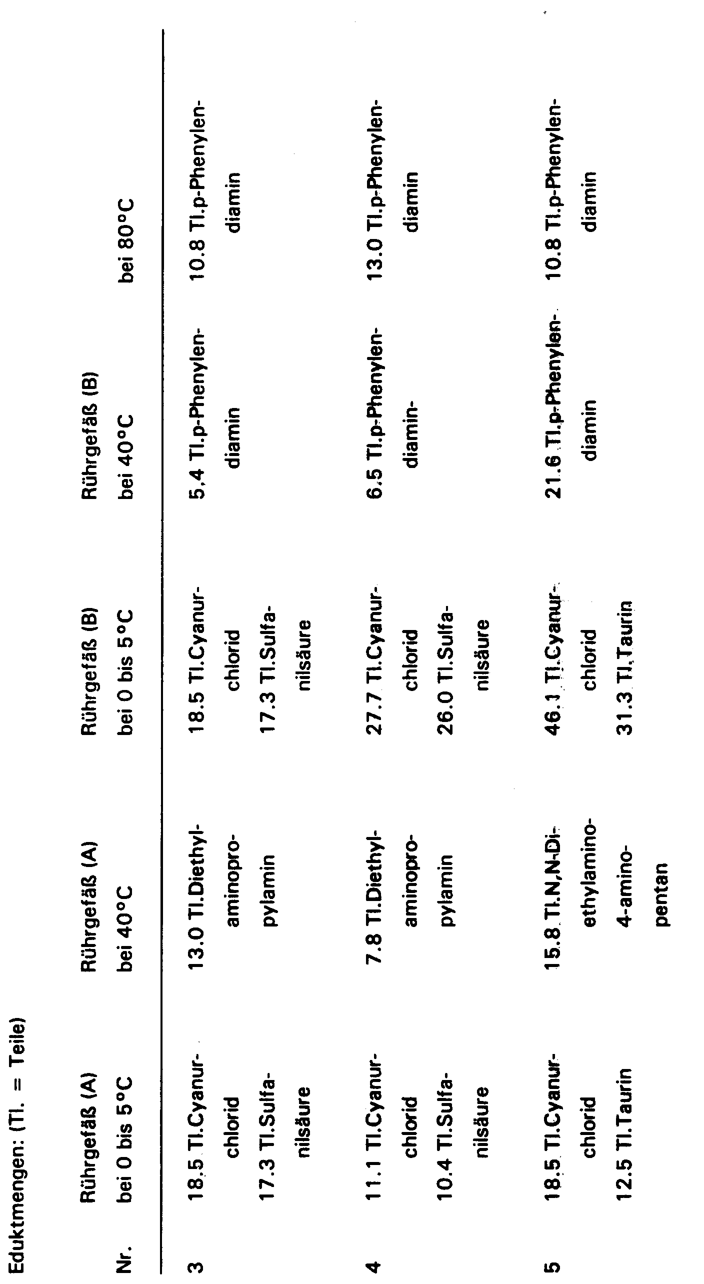

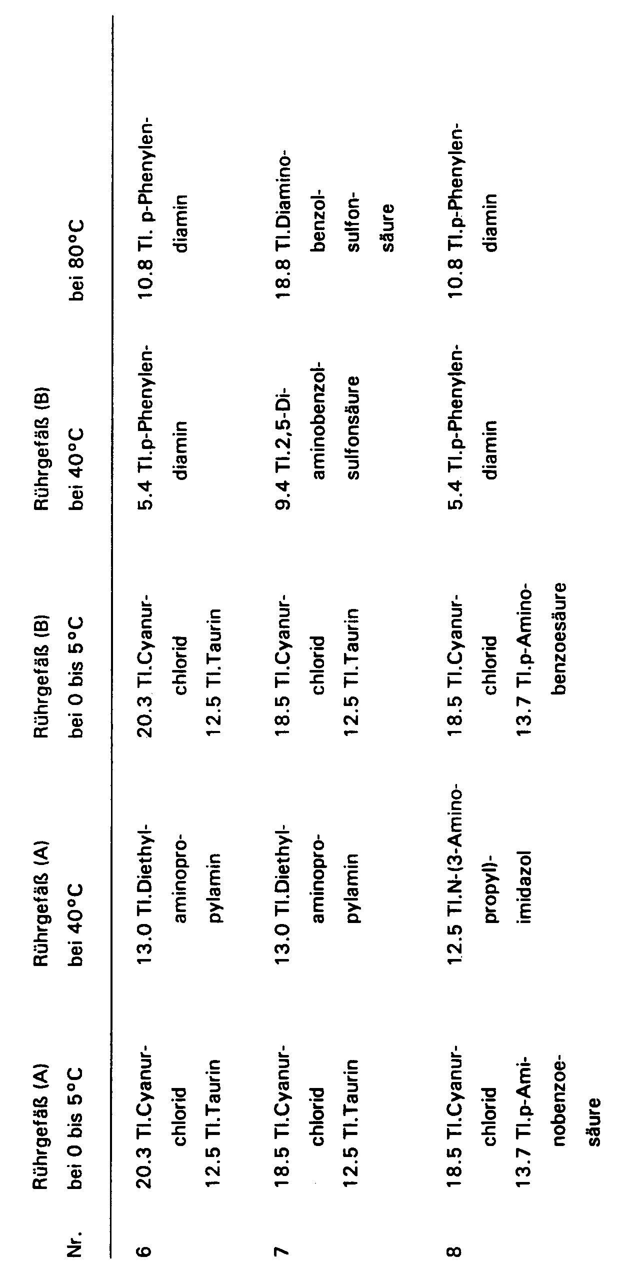

| Beispiel | IR (KBr, cm⁻¹) | | | | | | | | | | |
|---|---|---|---|---|---|---|---|---|---|---|---|
| 3 | 3400, | 3000, | 1630, | 1590, | 1550, | 1500, | 1410, | 1220, | 1160, | 1030, | 1000 |
| 4 | 3400, | 3000, | 1630, | 1590, | 1550, | 1490, | 1410, | 1220, | 1160, | 1030, | 1000 |
| 5 | 3300, | 3000, | 1630, | 1600, | 1560, | 1500, | 1410, | 1210, | 1160, | 1030 | |
| 6 | 3300, | 3000, | | | 1570, | 1500, | 1420, | 1210, | 1160, | 1030 | |

9) Zur Synthese der Verbindung der Formel IV mit
   X = -NH-CH₂-CH₂-CH₂-N(C₂H₅)₂, Y¹ = Y² = -NH-CH₂-CH₂-SO₃H,
   Z = -NH-C₆H₄-NH- und m = 2 wurden in einem Rührgefäß zu 400 Teilen einer Wasser-Eis-Mischung, die 0,4 Teile eines sulfonierten Fettsäurederivates und 36,9 Teile Cyanurchlorid enthielt, bei 0 bis 5°C 25,0 Teile Taurin gegeben und durch Zugabe von 0,2 normaler NaOH-Lösung der pH-Wert auf 6 bis 9 gehalten. Nach der Laugenzugabe wurde zwei Stunden bei 0 bis 5°C nachgerührt. Anschließend wurden bei ca. 40°C 13,0 Teile Diethylaminopropylamin zugegeben und durch Zugabe von 0,2 normaler NaOH-Lösung der pH-Wert auf 6 bis 9 gehalten. Nach der Laugenzugabe wurde eine Stunde bei ca. 40°C nachgerührt. Anschließend wurden bei ca. 80°C 16,2 Teile p-Phenylendiamin zugegeben und durch Zugabe von 0,2 normaler NaOH-Lösung der pH-Wert auf 6 bis 9 gehalten. Nach der Laugenzugabe wurde noch etwa eine Stunde bei 80°C und vier Stunden bei 95°C nachgerührt. Nachdem die Reaktionsmischung auf Raumtemperatur abgekühlt war, wurde mit konzentrierter Salzsäure ein pH-Wert von 1,5 eingestellt, der entstandene Niederschlag abfiltriert und getrocknet.
   IR (KBr, cm⁻¹) 3400, 3000, 1750, 1630, 1500, 1430, 1210, 1160, 1030
10) Herstellung der Aminsalzlösungen:
   Die in den Beispielen 1 bis 9 hergestellten, getrockneten Produkte wurden mit Wasser aufgeschlämmt und diese Aufschlämmungen mit einem Amin auf einen pH-Wert von 9 bis 10 eingestellt. Dabei löste sich die feste Substanz auf. Es ist auch möglich, den ungetrockneten Preßkuchen nach der Ausfällung mit Säure in den Beispielen 1 bis 9 direkt mit dem Amin zu versetzen, um eine klare Lösung herzustellen.

### Herstellungsbeispiele für Pigmentzubereitungen

I) In 1440 Teile VE-Wasser wurden 50 Teile Perylen-3,4,9,10-tetracarbonsäure-dianhydrid als feuchter Preßkuchen eingetragen. Nach Abkühlen der Suspension auf 0°C wurden innerhalb von 10 Minuten 75 Teile einer 40 %igen wäßrigen Monomethylaminlösung zugetropft. Es wurde noch 15 Minuten bei 0 bis 5°C nachgerührt und anschließend innerhalb von 15 Minuten eine Lösung von 28,5 Teilen wasserfreiem Calciumchlorid in 94,6 Teilen VE-Wasser zugegeben. Nach einer Stunde Rühren bei 0 bis 5°C wurden 26,0 Teile einer 21 %igen wäßrigen Lösung des Endprodukts aus Beispiel 5 zugegeben und die Suspension auf 80°C erwärmt und 2 Stunden bei dieser Temperatur gerührt. Anschließend wurde auf 50°C abgekühlt und mit 32,5 Teilen konzentrierter Ameisensäure ein pH-Wert von 8 bis 8,5 eingestellt. Nach 20 minütigem Rühren bei 50°C wurde die Pigmentzubereitung isoliert, mit Wasser salzfrei gewaschen und getrocknet. Man erhielt 54,8 Teile einer Zubereitung von C.I. Pigment Red 179.
II) In 1357 Teile VE-Wasser wurden 50 Teile Perylen-3,4,9,10-tetracarbonsäure-dianhydrid als feuchter Preßkuchen eingetragen. Nach Abkühlen der Suspension auf 0°C wurden innerhalb von 10 Minuten 93 Teile einer 32 %igen wäßrigen Monomethylaminlösung zugetropft. Es wurde 15 Minuten bei 0 bis 5°C gerührt und anschließend eine Lösung von 28,5 Teilen Calciumchlorid in 94,6 Teilen VE-Wasser zugegeben. Nach einstündigem Rühren bei 0 bis 5°C wurden 18,0 Teile einer 30 %igen wäßrigen Lösung des Endprodukts aus Beispiel 4 zugesetzt. Man erwärmte die Suspension auf 80°C und rührte 2 Stunden bei dieser Temperatur. Anschließend wurde auf 50°C abgekühlt und mit 29,9 Teilen konzentrierter Ameisensäure ein pH-Wert von 8 bis 8,5 eingestellt. Nach 30 minütigem Nachrühren bei 50°C wurde die Pigmentzubereitung isoliert, mit Wasser chloridfrei gewaschen und bei 80°C getrocknet. Man erhielt 58,8 Teile einer Zubereitung von C.I. Pigment Red 179.
III) Es wurde analog zu Beispiel I verfahren, nur daß anstelle von 26,0 Teilen der 21 %igen wäßrigen Lösung des Endprodukts aus Beispiel 5 23,7 Teile einer 22,5 %igen wäßrigen Lösung des Endprodukts aus Beispiel 6 eingesetzt wurden. Man erhielt 57,0 Teile einer Zubereitung von C.I. Pigment Red 179. Das Produkt enthielt 0,4 % Calcium-Ionen.
IV) Es wurde analog zu Beispiel II verfahren, nur daß anstelle von 18,0 Teilen der 30 %igen wäßrigen Lösung des Endprodukts aus Beispiel 4 9,0 Teile einer 30 %igen wäßrigen Lösung des Endprodukts aus Beispiel 8 eingesetzt wurden. Man erhielt 57,3 Teile einer Zubereitung von C.I. Pigment Red 179.
V) Es wurde analog zu Beispiel II verfahren, nur daß anstelle von 18,0 Teilen der 30 %igen wäßrigen Lösung des Endprodukts aus Beispiel 4 18,0 Teile einer 30 %igen wäßrigen Lösung des Endprodukts aus Beispiel 7 eingesetzt wurden. Man erhielt 54,1 Teile einer Zubereitung von C.I. Pigment Red 179.
VI) 40 Teile C.I. Pigment Violet 23 (hergestellt nach BIOS 960,75), welches noch 20 % des bei der Synthese anfallenden Salzes enthielt, wurden in ein Rührgefäß eingetragen, in dem 60 Teile 100 %iges Isobutanol und 2,5 Teile 98 %ige Ameisensäure vorgelegt waren. Während 15 Stunden Rühren bei 20 bis 25°C wurden 80 Teile Isobutanol (100 %) und 240 Teile VE-Wasser zugetropft. Anschließend wurde das Isobutanol bis zu einer Übergangstemperatur von 100°C mit Wasserdampf azeotrop abdestilliert. Der Destillationsrückstand wurde filtriert und der Filterkuchen mit Wasser salzfrei gewaschen. Man erhielt 64 Teile Pigmentpreßkuchen, der mit 250 Teilen VE-Wasser und 96 Teilen Isobutanol sowie 24,0 Teilen einer 15 %igen wäßrigen Lösung des Endprodukts aus Beispiel 6 in einem Autoklaven angerührt wurde. Die Suspension wurde 3 Stunden auf 125°C erhitzt. Man ließ auf 80°C abkühlen und destillierte das Isobutanol bis zu einer Übergangstemperatur von 100°C mit Wasserdampf azeotrop ab. Der Destillationsrückstand wurde filtriert, der Filterkuchen mit Wasser gewaschen und bei 80°C getrocknet. Man erhielt 32 Teile einer Zubereitung von C.I. Pigment Violet 23.
VII) 31,0 Teile C.I. Pigment Violet 23, welches nach BIOS 960,75 hergestellt wurde, wurden als feuchter Preßkuchen (40 %ig) in 96 Teile Isobutanol (100 %) und 195 Teile VE-Wasser eingetragen. Man gab 2,5 Teile konzentrierter Ameisensäure und 11,0 Teile NaCl hinzu und erhitzte 1 Stunde zum Rückfluß. Man ließ auf 60°C abkühlen und stellte mit verdünnter Natronlauge einen pH von 8 bis 9 ein. Anschließend fügte man 20,7 Teile einer 15 %igen wäßrigen Lösung des Endprodukts aus Beispiel 4 hinzu, rührte 30 Minuten nach und destillierte das Isobutanol mit Wasserdampf bis zu einer Übergangstemperatur von 100°C azeotrop ab. Der Destillationsrückstand wurde filtriert, der Filterkuchen mit Wasser chloridfrei gewaschen und bei 80°C getrocknet. Man erhielt 31,4 Teile einer Zubereitung von C.I. Pigment Violet 23.
VIII) 30,0 Teile C.I. Pigment Violet 23 wurden als feuchter Preßkuchen (Feststoffgehalt 40 %) in 95 Teile Isobutanol (100 %) und 195 Teile VE-Wasser eingetragen und 1 Stunde homogen verrührt. Man gab 13,7 Teile einer 27 %igen wäßrigen Lösung des Endprodukts aus Beispiel 6 hinzu, erhitzte zum Rückfluß und kochte 1 Stunde am Rückfluß. Anschließend destillierte man das Isobutanol mit Wasserdampf ab. Der Destillationsrückstand wurde filtriert, der Filterkuchen mit Wasser gewaschen und bei 80°C getrocknet. Man erhielt 30,5 Teile einer Zubereitung von C.I. Pigment Violet 23.
IX) 30 Teile C.I. Pigment Violet 23 wurden als feuchter Preßkuchen (Feststoffgehalt 40 %) in 280 Teile VE-Wasser eingetragen und 15 Minuten homogen verrührt. Man erhitzte auf 90 bis 95°C, gab 13,3 Teile einer 23 %igen wäßrigen Lösung des Endprodukts aus Beispiel 7 hinzu und ließ unter Rühren auf 30°C abkühlen. Nach Filtration und Trocknen bei 80°C erhielt man 29,5 Teile einer Zubereitung von C.I. Pigment Violet 23.
X) 44 Teile 2,9-Dimethylchinacridon (C.I. Pigment Red 122) wurden als feuchter Preßkuchen (Gehalt 24,5 %) in ein Rührgefäß eingetragen, in dem 270 Teile Isobutanol (100 %), 2,5 Teile Natriumhydroxid und 50 Teile VE-Wasser vorgelegt waren. Die Suspension wurde 15 Stunden bei etwa 20°C gerührt und anschließend 3 Stunden in einem Autoklaven auf 125°C erhitzt. Das Isobutanol wurde dann durch Wasserdampfdestillation entfernt und die wäßrige Suspension bei 50°C mit 25,1 Teilen einer 17,5 %igen wäßrigen Lösung des Endprodukts aus Beispiel 3 versetzt. Man rührte noch 3 Stunden bei 20 bis 25°C. Anschließend wurde filtriert, der Filterkuchen mit Wasser gewaschen und bei 80°C getrocknet. Man erhielt 43,4 Teile einer Zubereitung von C.I. Pigment Red 122.
XI) Man verfuhr wie in Beispiel VIII beschrieben, nur wurde die Pigmentzubereitung statt durch Filtration durch eine Sprühtrocknung isoliert. Man erhielt 23 Teile einer Zubereitung von C.I. Pigment Violet 23.
XII) 23 Teile eines Kupferphthalocyanins (C.I. Pigment Blue 15) wurden als feuchter Preßkuchen (Feststoffgehalt 23,5 %) in 250 Teile VE-Wasser eingetragen und über Nacht homogen verrührt. Man erhitzte dann auf 80°C, stellte mit Dimethylethanolamin auf pH 9 bis 9,5 und gab 10,2 Teile einer 22 %igen wäßrigen Lösung des Endprodukts aus Beispiel 6 zu. Man rührte noch 30 Minuten nach, wobei man auf Raumtemperatur abkühlen ließ. Anschließend wurde filtriert, der Filterkuchen mit Wasser gewaschen und bei 80°C getrocknet. Man erhielt 23,3 Teile einer Zubereitung von C.I. Pigment Blue 15.

### Anwendungsbeispiele für Pigmentdispersionen

A) 70 Teile eines gemahlenen Dolomits wurden mit einem Dissolver in 30 Teilen einer 22,6 %igen wäßrigen Lösung des Endprodukts aus Beispiel 6 45 Minuten dispergiert. Durch Zugabe von 6 Teilen VE-Wasser erhielt man eine dünnflüssige Dispersion, deren Rheologie mit der Note 4 bewertet wurde, während die Rheologie einer Paste, die ohne das Endprodukt aus Beispiel 6 erhalten wurde, gestockt war (Note 1).
B) 8,0 Teile eines oxidativ nachbehandelten Gasrußes mit einer spezifischen Oberfläche nach BET von 470 m²/g, welcher in wäßriger Suspension einen pH von 2,5 aufwies, wurden unter Zugabe von 1,8 Teilen einer 22 %igen wäßrigen Lösung des Endprodukts aus Beispiel 4 in 71,2 Teilen VE-Wasser mittels Dissolver 60 Minuten dispergiert. Man erhielt eine gut fließfähige Dispersion, deren Rheologie mit 4 bewertet wurde, während bei Weglassen der erfindungsgemäßen Verbindung eine leicht gestockte Dispersion (Note 2) resultierte.
C) 5,0 Teile desselben oxidativ nachbehandelten Gasrußes wie in Beispiel B wurden unter Zusatz von 2,2 Teilen einer wäßrigen Lösung des Endprodukts aus Beispiel 6 und 0,5 Teilen eines handelsüblichen Entschäumers in 42,5 Teilen einer 16 %igen wäßrigen Polyurethandispersion in Gegenwart von 50 Teilen Glasperlen (Durchmesser 1,0 mm) mit einem Dissolver 60 Minuten dispergiert. Man erhielt eine gut fließfähige, stabile Dispersion, deren Rheologie mit 4 bewertet wurde.
D) 50,0 Teile eines 40 %igen feuchten Preßkuchens von C.I. Pigment Violet 23 wurden unter Zusatz von 8,5 Teilen einer 22 %igen wäßrigen Lösung des Endprodukts aus Beispiel 6 mit 41,5 Teilen VE-Wasser versetzt. Man gab 100 Teile Glasperlen (Durchmesser 1,0 mm) zu und dispergierte 60 Minuten in einer Laborperlmühle. Man erhielt nach Absieben der Perlen eine gut fließfähige Dispersion von C.I. Pigment Violet 23, deren Rheologie mit 5 benotet wurde.
E) 6,0 Teile Dimethylperylimid (C.I. Pigment Red 179), 53,4 Teile einer 16 %igen wäßrigen Polyurethandispersion und 0,6 Teile eines handelsüblichen Entschäumers wurden zusammen mit 2,8 Teilen einer 22 %igen wäßrigen Lösung des Endprodukts aus Beispiel 6 15 Minuten am Dissolver homogenisiert. Nach Zugabe von 100 Teilen Glasperlen (Durchmesser 1,0 mm) wurde 60 Minuten auf einer Laborperlmühle bei 40°C dispergiert. Man erhielt eine gut fließfähige Paste (Rheologie 5), die nach Verdünnen ("Auflacken") mit 119,2 Teilen eines 18 %igen wäßrigen Polyurethanharzes und 20,8 Teilen VE-Wasser einen merklich farbstärkeren Überzug lieferte im Vergleich zu einem Pigment (Vergleich), das ohne Zusatz des erfindungsgemäßen Stoffes dispergiert wurde.

| | VT 3 % P | 1 : 10 TiO₂ | 50 : 50 Met | Visk (s) |
|---|---|---|---|---|
| Vergleich | --- | --- | --- | 2,5 |
| Beispiel E | wenig transparenter | wesentlich farbstärker | deutlich farbstärker | 1,0 |

F) 12,0 Teile C.I. Pigment Violet 23, 47,5 Teile eines 25 %igen wäßrigen Polyurethanharzes und 0,5 Teile eines handelsüblichen Entschäumers wurden nach Zusatz von 5,5 Teilen einer 22 %igen wäßrigen Lösung des Endprodukts aus Beispiel 6 15 Minuten am Dissolver homogen verrührt und anschließend nach Zusatz von 90 Teilen Glasperlen (Durchmesser 1,0 mm) 60 Minuten auf einer Minisandmühle gemahlen. Nach Absieben der Perlen wurde mit 330,1 Teilen einer 15,3 %igen wäßrigen Polyurethandispersion und 9,9 Teilen VE-Wasser aufgelackt. Man erhielt mit diesem Lack einen deutlich farbstärkeren und glänzenderen Überzug im Vergleich zu einer Anreibung ohne die erfindungsgemäße Verbindung (Vergleich).

| | VT 3 % P | 1 : 10 TiO₂ | 50 : 50 Met | Glanz |
|---|---|---|---|---|
| Vergleich | --- | --- | --- | 15 |
| Beispiel F | gleich transparent | wesentlich farbstärker | wesentlich farbstärker | 56 |

G) 20,0 Teile der Pigmentzubereitung von C.I. Pigment Red 179 aus Beispiel II, 44,0 Teile VE-Wasser und 36,0 Teile eines 27 %igen wäßrigen Polyacrylatharzes wurden 15 Minuten mittels Dissolver homogenisiert. Diese Suspension wurde nach Zusatz von 660 Teilen Zirkondioxid-Perlen (Durchmesser 1,0 bis 1,6 mm) und weiteren 33,3 Teilen VE-Wasser 90 Minuten in einer 0,5 Ltr.-Laborperlmühle dispergiert. Man erhielt eine 15 %ige Pigmentpaste mit sehr guter Fließfähigkeit und Lagerstabilität. Man verdünnte diese Paste mit einer wäßrigen Polyurethandispersion auf 5 % Pigmentgehalt und erzielte damit im Vergleich zu einem Pigment, welches nach der US-PS 4 496 731, Beispiel 5, hergestellt wurde, einen merklich farbstärkeren und transparenteren Überzug.
H) 6,0 Teile der Pigmentzubereitung von C.I. Pigment Red 179 aus Beispiel III, 0,5 Teile eines handelsüblichen Entschäumers und 53,5 Teile einer 30 %igen wäßrigen Polyesterdispersion wurden 15 Minuten mit einem Dissolver vorgemischt und nach Zusatz von 100 Teilen Glasperlen (Durchmesser 1,0 mm) 60 Minuten auf einer Minisandmühle bei 50°C dispergiert. Man erhielt eine 10 %ige Pigmentpaste mit guter Fließfähigkeit. Man lackte diese Paste mit 90 Teilen einer 26 %igen wäßrigen Polyester/Melaminharzdispersion auf einen Pigmentgehalt von 3 % auf und erzielte damit gegenüber einem nach der US-PS 4 496 731 hergestellten Pigment einen merklich farbstärkeren und transparenteren Überzug.
J) 6,0 Teile der Zubereitung von C.I. Pigment Red 179 aus Beispiel III, 53,4 Teile einer 16 %igen wäßrigen Polyurethandispersion und 0,6 Teile eines handelsüblichen Entschäumers wurden 15 Minuten am Dissolver homogenisiert. Nach Zugabe von 100 Teilen Glasperlen (Durchmesser 1,0 mm) wurde 60 Minuten auf einer Minisandmühle bei 40°C dispergiert. Man erhielt eine im Vergleich zu einem Handelspigment besser fließfähige Paste, die nach Auflacken mit 119,2 Teilen eines 18 %igen wäßrigen Polyurethanharzes und 20,8 Teilen VE-Wasser einen deutlich transparenteren und farbstärkeren Überzug lieferte im Vergleich zu Pigmenten, die gemäß den US-PS 4 496 731 oder US-PS 4 189 582 hergestellt wurden, und zu Pigmentzubereitungen, die mit Additiven belegt sind, wie sie in den US-PS 3 580 827, Beispiel 5, und US-PS 4 314 001, Beispiel 3, beschrieben sind.
Mit einem Rotationsviskosimeter wurden Fließkurven aufgenommen (Bedingungen: 22°C, Ruhen 10 Minuten, Steigen auf maximale Drehfrequenz in 2 Minuten, Halten 1 Minute und Absenken der Drehfrequenz auf Null in 2 Minuten), um das rheologische Verhalten der erfindungsgemäßen Pigmentzubereitung aus Beispiel J mit dem eines Pigments, das gemäß der US-PS 4 496 731, Beispiel 5, hergestellt wurde, zu vergleichen. Bei der erfindungsgemäßen Pigmentzubereitung zeigte sich dabei eine Vikositätserniedrigung um ca. 25 % gegenüber dem Vergleich.
Die coloristische Prüfung ergab folgende Unterschiede:

| | VT 3 % P | 1 : 10 TiO₂ | 50 : 50 Met | Glanz |
|---|---|---|---|---|
| Pigment mit Additiv gemäß US-PS 4 314 001 | --- | --- | --- | 22 |
| Beispiel J | wesentlich transparenter | wesentlich farbstärker | wesentlich farbstärker | 61 |

| | VT 3 % P | 1 : 10 TiO₂ | 50 : 50 Met |
|---|---|---|---|
| Pigment nach US-PS 4 496 731 (Beispiel 5) | --- | --- | --- |
| Beispiel J | wesentlich transparenter | deutlich farbstärker | deutlich farbstärker |

| | VT 3 % P | 1 : 10 TiO₂ | 50 : 50 Met |
|---|---|---|---|
| Pigment nach US-PS 4 189 582 (Beispiel 2) | --- | --- | --- |
| Beispiel J | deutlich transparenter | merklich farbstärker | etwas farbstärker |

Ein Vergleich der Bunttonwinkel (gemessen nach DIN 5033) verschiedener Pigmentzubereitungen ergab folgende Werte: Pigmentzubereitung mit der erfindungsgemäßen Verbindung aus Beispiel J: 27,5 Grad, Pigmentzubereitung nach US-PS 4 189 582, Beispiel 2: 23 Grad, Pigmentzubereitung nach US-PS 4 496 731, Beispiel 5: 21 Grad.
K) 12,0 Teile der Pigmentzubereitung von C.I. Pigment Violet 23 aus Beispiel VII, 0,5 Teile eines handelsüblichen Entschäumers und 47,5 Teile einer 20 %igen wäßrigen Polyurethanharzlösung wurden nach Zugabe von 100 Teilen Glasperlen (Durchmesser 1,0 mm) 60 Minuten auf einer Minisandmühle bei 40°C dispergiert. Man erhielt eine 20 %ige Pigmentpaste mit sehr guter Fließfähigkeit. Man lackte diese Paste mit 330,1 Teilen einer 15,3 %igen wäßrigen Polyurethandispersion und 9,9 Teilen VE-Wasser auf einen Pigmentgehalt von 3 % auf und erzielte damit gegenüber einem unbehandelten Pigment (Vergleich) sowie einem Pigment, das nach der US-PS 4 253 839, Beispiel 2, hergestellt wurde, einen wesentlich transparenteren und farbstärkeren Überzug mit höherem Glanz.

| | VT 3 % P | 1 : 10 TiO₂ | 50 : 50 Met | Visk (s) | Glanz |
|---|---|---|---|---|---|
| Vergleich | --- | --- | --- | 3,5 | 30 |
| Beispiel K | wesentlich transparenter | bedeutend farbstärker | bedeutend farbstärker | 4,9 | 71 |

| | VT 3 % P | 1 : 10 TiO₂ | 50 : 50 Met | Visk (s) | Glanz |
|---|---|---|---|---|---|
| Pigment nach US-PS 4 253 839 | | --- | --- | 8,9 | 30 |
| Beispiel K | deutlich transparenter | bedeutend farbstärker | wesentlich farbstärker | 4,9 | 71 |

### Formelschema

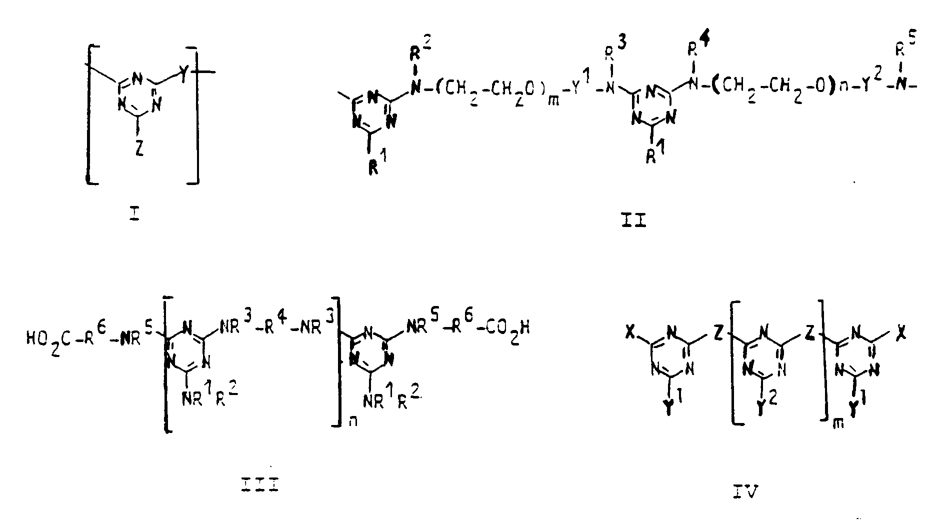

-A¹-D¹-NR¹R² (IVa),

-NH-C₂-C₃-Alkylen-A⁵-C₂-C₃-Alkylen-NH- (IVf),

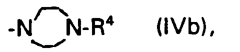

-NR⁸-D¹-NR¹R² (IVg),

-A³-D²-E¹ (IVc),

-NR⁹-D²-E¹ (IVh),

-A⁴-Z*-A⁴- (IVd),

-NR¹⁰-Z*-NR¹⁰- (IVi),

-NH-C₆H₄-A⁵-C₆H₄-NH- (IVe),

-NH-C₂-C₃-Alkylen-Z**-C₂-C₃-Alkylen-NH- (IVj)

### Reaktionsschemata

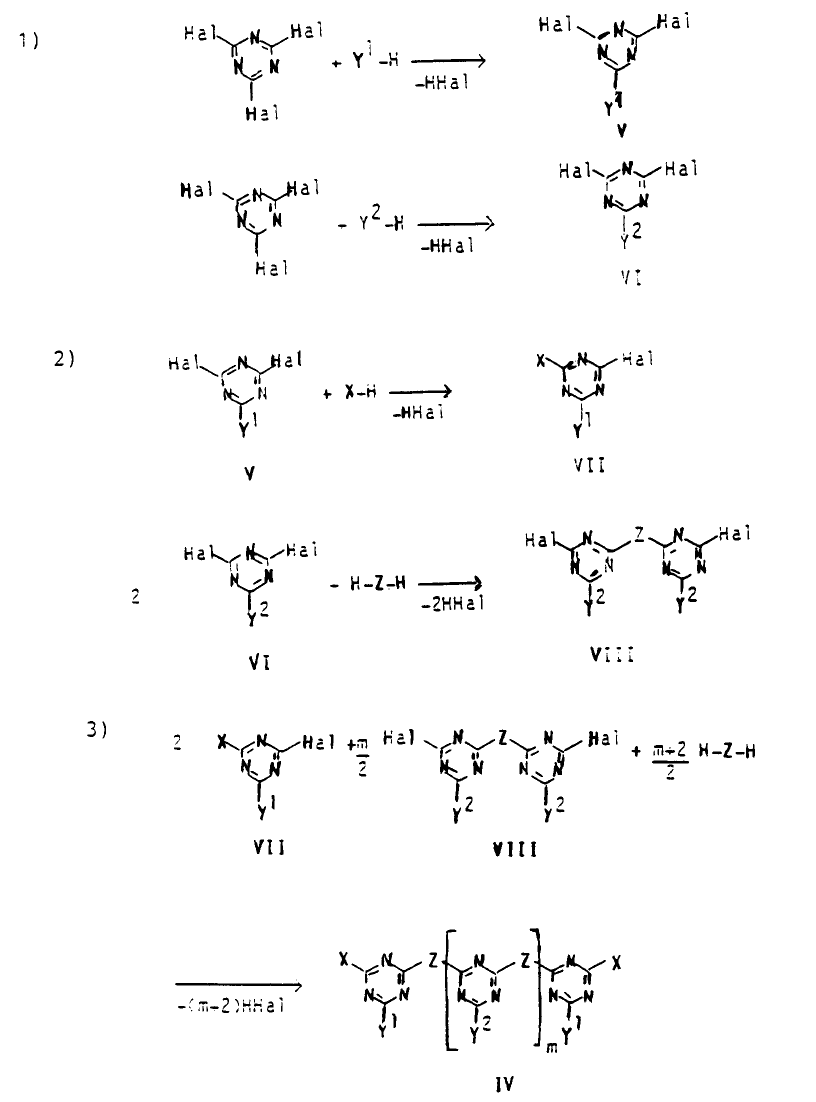

## Patentansprüche (Patentansprüche für folgende(n) Vertragsstaat(en): CH, DE, DK, FR, GB, IT, LI, NL)

1. Triazinverbindungen der allgemeinen Formel IV 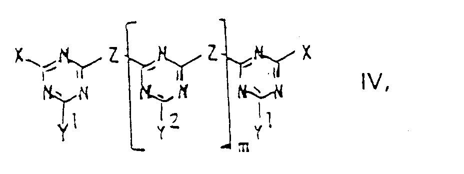 wobei
X eine Gruppe der Formel IVa
-A¹-D¹-NR¹R² IVa
darstellt, in der A¹ ein Brückenglied NR³ ist, worin R³ Wasserstoff oder einen C₁-C₂₂-Alkylrest oder einen C₃-C₂₂-Alkenylrest darstellt, der gegebenenfalls durch eine OH-Gruppe substituiert ist,
D¹ eine Arylengruppe oder eine verzweigte oder unverzweigte C₂-C₁₂-Alkylengruppe ist, welche gegebenenfalls durch ein oder mehrere Brückenglieder aus der Gruppe A² unterbrochen ist, und A² unabhängig von A¹ dieselbe Gruppe von Substituenten wie A¹ bedeutet,
und R¹ und R² unabhängig voneinander verzweigte oder unverzweigte C₁-C₂₀-Alkylgruppen oder C₃-C₂₀-Alkenylgruppen sind, oder in der R¹ und R² zusammen mit dem Stickstoffatom einen ungesättigten oder gesättigten fünf- oder sechsgliedrigen Ring bilden, welcher gegebenenfalls zusätzlich ein oder zwei Atome Stickstoff, Sauerstoff und/oder Schwefel als weitere Heteroatome im Ring enthält,
oder wobei X eine Gruppe der Formel IVb 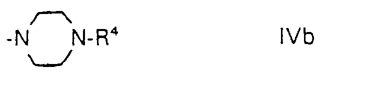 darstellt, worin R⁴ C₁-C₆-Alkyl bedeutet, sowie
Y¹ und Y² unabhängig voneinander jeweils eine Gruppe der Formel IVc
-A³-D²-E¹ IVc
darstellen,
in der A³ unabhängig von A¹ ist und ein Brückenglied aus derselben Gruppe von Substituenten wie A¹ bedeutet,
D² unabhängig von D¹ ist und dieselbe Gruppe von Substituenten wie D¹, CH₂ oder eine direkte Bindung bedeutet,
und E¹ COOM, SO₃M, OSO₃M oder OPO₃M₂ ist, worin M Wasserstoff, Metall oder die stöchiometrische Menge eines Erdalkalimetalls oder ein Ammoniumion ist, welches gegebenenfalls durch aliphatische, aromatische und araliphatische Reste substituiert ist, und
Z eine Gruppe der Formel IVd
-A⁴-Z*-A⁴- IVd
darstellt, in der A⁴ unabhängig von A¹ ist und ein Brückenglied aus derselben Gruppe von Substituenten wie A¹ bedeutet und
Z* die Gruppe CO, verzweigtes oder unverzweigtes C₂-C₂₅-Alkylen,
C₅-C₆-Cycloalkylen oder C₆-C₁₄-Arylen ist, welches gegebenenfalls durch COOM, SO₃M, Cl, OH oder Alkoxygruppen substituiert ist, wobei M die obengenannten Bedeutungen hat, und wobei in den cyclischen Verbindungen gegebenenfalls die Kohlenstoffatome teilweise durch die Heteroatome Stickstoff, Sauerstoff und/oder Schwefel ersetzt sind,
oder wobei Z ein Brückenglied der allgemeinen Formeln IVe und IVf
-NH-C₆H₄-A⁵-C₆H₄-NH- IVe
-NH-C₂-C₃-Alkylen-A⁵-C₂-C₃-Alkylen-NH- IVf
darstellt, in denen A⁵ eine Gruppe der Formeln CR⁵R⁶, NR⁷, O, SO, SO₂ und CO ist, wobei R⁵ und R⁶ unabhängig voneinander verzweigte oder unverzweigte C₁-C₄-Alkylgruppen sowie Wasserstoff sein können und wobei R⁷ unabhängig von R³ dieselbe Gruppe von Substituenten wie R³ bedeutet,
oder Z eine beliebige Kombination der Gruppen der Formeln IVd, IVe und/oder IVf ist, und
m eine ganze Zahl von 1 bis 100 bedeutet.

2. Triazinverbindungen der allgemeinen Formel IV gemäß Anspruch 1, wobei
X eine Gruppe der Formel IVa
-A¹-D¹-NR¹R² IVa
darstellt, in der
A¹ ein Brückenglied NR³ ist, worin R³ Wasserstoff oder einen C₁-C₂₂-Alkylrest oder einen C₃-C₂₂-Alkenylrest darstellt, der gegebenenfalls durch eine OH-Gruppe substituiert ist,
D¹ eine Arylengruppe oder eine verzweigte oder unverzweigte C₂-C₁₂-Alkylengruppe ist, welche gegebenenfalls durch ein oder mehrere Brückenglieder aus der Gruppe A² unterbrochen ist, und A² unabhängig von A¹ dieselbe Gruppe von Substituenten wie A¹ bedeutet, und
R¹ und R² unabhängig voneinander verzweigte oder unverzweigte C₁-C₂₀-Alkylgruppen oder C₃-C₂₀-Alkenylgruppen sind, oder in der R¹ und R² zusammen mit dem Stickstoffatom einen ungesättigten oder gesättigten fünf- oder sechsgliedrigen Ring bilden, welcher gegebenenfalls zusätzlich ein oder zwei Atome Stickstoff und/oder Sauerstoff als weitere Heteroatome im Ring enthält, oder wobei X eine Gruppe der Formel IVb 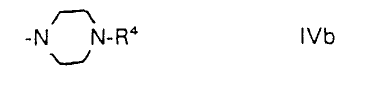 darstellt, worin R⁴ C₁-C₆-Alkyl bedeutet, sowie
Y¹ und Y² unabhängig voneinander jeweils eine Gruppe der Formel IVc
-A³-D²-E¹ IVc
darstellen, in der A³ unabhängig von A¹ ist und ein Brückenglied aus derselben Gruppe von Substituenten wie A¹ bedeutet, D² unabhängig von D¹ ist und dieselbe Gruppe von Substituenten wie D¹, CH₂ oder eine direkte Bindung bedeutet, und E¹ COOM oder SO₃M ist, worin M Wasserstoff, Alkalimetall oder die stöchiometrische Menge eines Erdalkalimetalls, oder ein Ammoniumion, welches gegebenenfalls durch aliphatische, aromatische und araliphatische Reste substituiert ist, und
Z eine Gruppe der Formel IVd
-A⁴-Z*-A⁴- IVd
darstellt, in der A⁴ unabhängig von A¹ ist und ein Brückenglied aus derselben Gruppe von Substituenten wie A¹ bedeutet, und Z* die Gruppe CO, verzweigtes oder unverzweigtes C₂-C₂₅-Alkylen, C₅-C₆-Cycloalkylen oder Phenylen ist, welches gegebenenfalls durch COOM oder SO₃M substituiert ist, wobei M die obengenannten Bedeutungen hat, und wobei in den cyclischen Verbindungen gegebenenfalls die Kohlenstoffatome teilweise durch die Heteroatome Stickstoff und/oder Sauerstoff ersetzt sind, oder Z eine Kombination der Gruppen der Formel IVd darstellt, und
m eine ganze Zahl von 1 bis 100 bedeutet.

3. Triazinverbindungen der allgemeinen Formel IV gemäß Anspruch 1, wobei
X eine Gruppe der Formel IVg
-NR⁸-D¹-NR¹R² IVg
darstellt, in der R⁸ Methyl oder Wasserstoff ist, und D¹ eine verzweigte oder unverzweigte C₂-C₆-Alkylengruppe oder eine Phenylengruppe ist, und R¹ und R² unabhängig voneinander verzweigte oder unverzweigte C₁-C₆-Alkylgruppen sind, oder in der R¹ und R² zusammen mit dem Stickstoffatom einen ungesättigten oder gesättigten fünf- oder sechsgliedrigen Ring bilden, welcher gegebenenfalls zusätzlich ein oder zwei Atome Stickstoff und/oder Sauerstoff als weitere Heteroatome im Ring enthält, sowie
Y¹ und Y² unabhängig voneinander jeweils eine Gruppe der Formel IVh
-NR⁹-D²-E¹ IVh
darstellen, in der R⁹ unabhängig von R⁸ ist und ein Substituent aus derselben Gruppe wie R⁸ bedeutet, und D² unabhängig von D¹ ist und dieselbe Gruppe von Substituenten wie D¹, CH₂ oder eine direkte Bindung bedeutet, und E¹ COOM oder SO₃M ist, worin M Wasserstoff, Alkalimetall oder die stöchiometrische Menge eines Erdalkalimetalls, oder ein Ammoniumion, welches gegebenenfalls durch aliphatische, aromatische und araliphatische Reste substituiert ist, und
Z eine Gruppe der Formel IVi
-NR¹⁰-Z*-NR¹⁰- IVi
darstellt, in der R¹⁰ Methyl oder Wasserstoff ist, und Z* die Gruppe C₂-C₆-Alkylen oder Phenylen ist, welches gegebenenfalls durch COOM oder SO₃M substituiert ist, wobei M die obengenannten Bedeutungen hat, oder wobei Z eine Gruppe der Formel IVj
-NH-C₂-C₃-Alkylen-Z**-C₂-C₃-Alkylen-NH- IVj
darstellt, in der Z** die Gruppe N-C₁-C₂₀-Alkyl, NH oder O bedeutet, und
m eine ganze Zahl von 1 bis 30 bedeutet.

4. Verfahren zur Herstellung einer Verbindung nach Anspruch 1, dadurch gekennzeichnet, daß man in getrennten Behältern zunächst eine Verbindung der Formel Y¹-H (erster Reaktionsansatz) und Y²-H (zweiter Reaktionsansatz) jeweils in wäßriger Lösung mit Cyanurhalogenid bei einer Temperatur von 0 bis 10°C umsetzt, anschließend das im ersten Reaktionsansatz entstandene Zwischenprodukt mit einer Verbindung der Formel X-H und das im zweiten Reaktionsansatz entstandene Zwischenprodukt mit einer Verbindung der Formel ZH₂ jeweils bei einer Temperatur von 20 bis 60°C umsetzt, danach die beiden Reaktionsansätze zusammengibt und mit 0,1 bis 0,6 Mol ZH₂ pro Mol eingesetztem Cyanurhalogenid bei einer Temperatur von 70 bis 150°C umsetzt.

5. Verfahren nach Anspruch 4, dadurch gekennzeichnet, daß die gesamte Menge an ZH₂ bereits vor dem Zusammengeben der beiden Reaktionsansätze im zweiten Reaktionsansatz vorhanden ist.

6. Verwendung der Verbindungen nach mindestens einem der Ansprüche 1 bis 3 als grenzflächenaktive Mittel.

7. Verwendung der Verbindungen nach mindestens einem der Ansprüche 1 bis 3 als Dispergier-, Emulgier- oder Verteilungsmittel von Feststoffen in wäßrigen Medien.

8. Verwendung der Verbindungen nach mindestens einem der Ansprüche 1 bis 3 als Dispergiermittel von Feststoffen, vorzugsweise von Mineralien, Pflanzenschutzmitteln und Farbstoffen, von Pigmenten und optischen Aufhellern.

9. Pigmentdispersion, gekennzeichnet im wesentlichen durch den Gehalt an mindestens einer der Verbindungen der Formel IV gemäß Anspruch 1 und mindestens einem anorganischen und/oder organischen Pigment.

10. Verwendung der Pigmentdispersion gemäß Anspruch 9 zur Pigmentierung von wäßrigen oder wasserverdünnbaren Einbrennlacksystemen oder wäßrigen Bindemitteln.

11. Pigmentzubereitung, gekennzeichnet im wesentlichen durch den Gehalt an
a) 99,5 bis 50 Gew.-% mindestens eines organischen Pigments,
b) 0,5 bis 30 Gew.-% mindestens einer Verbindung der Formel IV gemäß Anspruch 1 und
c) 0 bis 20 Gew.-% an weiteren üblichen Zusatzstoffen.

12. Pigmentzubereitung, gekennzeichnet im wesentlichen durch den Gehalt an
a) 97 bis 70 Gew.-% mindestens eines polycyclischen organischen Pigments,
b) 3 bis 20 Gew.-% mindestens einer Verbindung der Formel IV gemäß Anspruch 1 und
c) 0 bis 10 Gew.-% an weiteren üblichen Zusatzstoffen.

13. Verfahren zur Herstellung einer Pigmentzubereitung gemäß Anspruch 11 oder 12, dadurch gekennzeichnet daß die Oberfläche der Pigmente mit mindestens einer Verbindung der Formel IV beschichtet wird.

14. Verwendung einer Pigmentzubereitung gemäß Anspruch 11 oder 12 zur Pigmentierung von wäßrigen oder wasserverdünnbaren Einbrennlacksystemen oder wäßrigen Bindemitteln.

## Patentansprüche (Patentansprüche für folgende(n) Vertragsstaat(en): ES)

1. Verfahren zur Herstellung einer Triazinverbindung der allgemeinen Formel IV 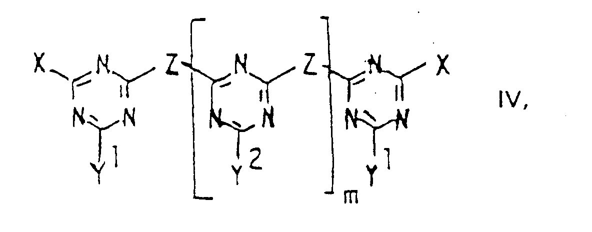 wobei
X eine Gruppe der Formel IVa
-A¹-D¹-NR¹R² IVa
darstellt, in der A¹ ein Brückenglied NR³ ist, worin R³ Wasserstoff oder einen C₁-C₂₂-Alkylrest oder einen C₃-C₂₂-Alkenylrest darstellt, der gegebenenfalls durch eine OH-Gruppe substituiert ist,
D¹ eine Arylengruppe oder eine verzweigte oder unverzweigte C₂-C₁₂-Alkylengruppe ist, welche gegebenenfalls durch ein oder mehrere Brückenglieder aus der Gruppe A² unterbrochen ist, und A² unabhängig von A¹ dieselbe Gruppe von Substituenten wie A¹ bedeutet,
und R¹ und R² unabhängig voneinander verzweigte oder unverzweigte C₁-C₂₀-Alkylgruppen oder C₃-C₂₀-Alkenylgruppen sind, oder in der R¹ und R² zusammen mit dem Stickstoffatom einen ungesättigten oder gesättigten fünf- oder sechsgliedrigen Ring bilden, welcher gegebenenfalls zusätzlich ein oder zwei Atome Stickstoff, Sauerstoff und/oder Schwefel als weitere Heteroatome im Ring enthält,
oder wobei X eine Gruppe der Formel IVb 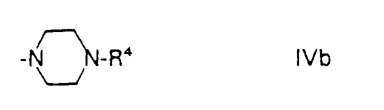 darstellt, worin R⁴ C₁-C₆-Alkyl bedeutet, sowie
Y¹ und Y² unabhängig voneinander jeweils eine Gruppe der Formel IVc
-A³-D²-E¹ IVc
darstellen,
in der A³ unabhängig von A¹ ist und ein Brückenglied aus derselben Gruppe von Substituenten wie A¹ bedeutet,
D² unabhängig von D¹ ist und dieselbe Gruppe von Substituenten wie D¹, CH₂ oder eine direkte Bindung bedeutet,
und E¹ COOM, SO₃M, OSO₃M oder OPO₃M₂ ist, worin M Wasserstoff, Metall oder die stöchiometrische Menge eines Erdalkalimetalls oder ein Ammoniumion ist, welches gegebenenfalls durch aliphatische, aromatische und araliphatische Reste substituiert ist, und
Z eine Gruppe der Formel IVd
-A⁴-Z*-A⁴- IVd
darstellt, in der A⁴ unabhängig von A¹ ist und ein Brückenglied aus derselben Gruppe von Substituenten wie A¹ bedeutet und
Z* die Gruppe CO, verzweigtes oder unverzweigtes C₂-C₂₅-Alkylen, C₅-C₆-Cycloalkylen oder C₆-C₁₄-Arylen ist, welches gegebenenfalls durch COOM, SO₃M, Cl, OH oder Alkoxygruppen substituiert ist, wobei M die obengenannten Bedeutungen hat, und wobei in den cyclischen Verbindungen gegebenenfalls die Kohlenstoffatome teilweise durch die Heteroatome Stickstoff, Sauerstoff und/oder Schwefel ersetzt sind,
oder wobei Z ein Brückenglied der allgemeinen Formeln IVe und IVf
-NH-C₆H₄-A⁵-C₆H₄-NH- IVe
-NH-C₂-C₃-Alkylen-A⁵-C₂-C₃-Alkylen-NH- IVf
darstellt, in denen A⁵ eine Gruppe der Formeln CR⁵R⁶, NR⁷, O, SO, SO₂ und CO ist, wobei R⁵ und R⁶ unabhängig voneinander verzweigte oder unverzweigte C₁-C₄-Alkylgruppen sowie Wasserstoff sein können und wobei R⁷ unabhängig von R³ dieselbe Gruppe von Substituenten wie R³ bedeutet, oder Z eine beliebige Kombination der Gruppen der Formeln IVd, IVe und/oder IVf ist, und
m eine ganze Zahl von 1 bis 100 bedeutet,
dadurch gekennzeichnet, daß man in getrennten Behältern zunächst eine Verbindung der Formel Y¹-H (erster Reaktionsansatz) und Y²-H (zweiter Reaktionsansatz) jeweils in wäßriger Lösung mit Cyanurhalogenid bei einer Temperatur von 0 bis 10°C umsetzt, anschließend das im ersten Reaktionsansatz entstandene Zwischenprodukt mit einer Verbindung der Formel X-H und das im zweiten Reaktionsansatz entstandene Zwischenprodukt mit einer Verbindung der Formel ZH₂ jeweils bei einer Temperatur von 20 bis 60°C umsetzt, danach die beiden Reaktionsansätze zusammengibt und mit 0,1 bis 0,6 Mol ZH₂ pro Mol eingesetztem Cyanurhalogenid bei einer Temperatur von 70 bis 150°C umsetzt.

2. Verfahren gemäß Anspruch 1, dadurch gekennzeichnet, daß
X eine Gruppe der Formel IVa
-A¹-D¹-NR¹R² IVa
darstellt, in der
A¹ ein Brückenglied NR³ ist, worin R³ Wasserstoff oder einen C₁-C₂₂-Alkylrest oder einen C₃-C₂₂-Alkenylrest darstellt, der gegebenenfalls durch eine OH-Gruppe substituiert ist,
D¹ eine Arylengruppe oder eine verzweigte oder unverzweigte C₂-C₁₂-Alkylengruppe ist, welche gegebenenfalls durch ein oder mehrere Brückenglieder aus der Gruppe A² unterbrochen ist, und A² unabhängig von A¹ dieselbe Gruppe von Substituenten wie A¹ bedeutet, und
R¹ und R² unabhängig voneinander verzweigte oder unverzweigte C₁-C₂₀-Alkylgruppen oder C₃-C₂₀-Alkenylgruppen sind, oder in der R¹ und R² zusammen mit dem Stickstoffatom einen ungesättigten oder gesättigten fünf- oder sechsgliedrigen Ring bilden, welcher gegebenenfalls zusätzlich ein oder zwei Atome Stickstoff und/oder Sauerstoff als weitere Heteroatome im Ring enthält, oder wobei X eine Gruppe der Formel IVb 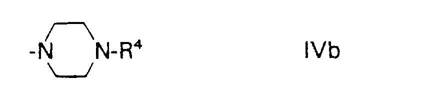 darstellt, worin R⁴ C₁-C₆-Alkyl bedeutet, sowie
Y¹ und Y² unabhängig voneinander jeweils eine Gruppe der Formel IVc
-A³-D²-E¹ IVc
darstellen, in der A³ unabhängig von A¹ ist und ein Brückenglied aus derselben Gruppe von Substituenten wie A¹ bedeutet, D² unabhängig von D¹ ist und dieselbe Gruppe von Substituenten wie D¹, CH₂ oder eine direkte Bindung bedeutet, und E¹ COOM oder SO₃M ist, worin M Wasserstoff, Alkalimetall oder die stöchiometrische Menge eines Erdalkalimetalls, oder ein Ammoniumion, welches gegebenenfalls durch aliphatische, aromatische und araliphatische Reste substituiert ist, und
Z eine Gruppe der Formel IVd
-A⁴-Z*-A⁴- IVd
darstellt, in der A⁴ unabhängig von A¹ ist und ein Brückenglied aus derselben Gruppe von Substituenten wie A¹ bedeutet, und Z* die Gruppe CO, verzweigtes oder unverzweigtes C₂-C₂₅-Alkylen, C₅-C₆-Cycloalkylen oder Phenylen ist, welches gegebenenfalls durch COOM oder SO₃M substituiert ist, wobei M die obengenannten Bedeutungen hat, und wobei in den cyclischen Verbindungen gegebenenfalls die Kohlenstoffatome teilweise durch die Heteroatome Stickstoff und/oder Sauerstoff ersetzt sind, oder Z eine Kombination der Gruppen der Formel IVd darstellt, und
m eine ganze Zahl von 1 bis 100 bedeutet.

3. Verfahren gemäß Anspruch 1, dadurch gekennzeichnet, daß
X eine Gruppe der Formel IVg
-NR⁸-D¹-NR¹R² IVg
darstellt, in der R⁸ Methyl oder Wasserstoff ist, und D¹ eine verzweigte oder unverzweigte C₂-C₆-Alkylengruppe oder eine Phenylengruppe ist, und R¹ und R² unabhängig voneinander verzweigte oder unverzweigte C₁-C₆-Alkylgruppen sind, oder in der R¹ und R² zusammen mit dem Stickstoffatom einen ungesättigten oder gesättigten fünf- oder sechsgliedrigen Ring bilden, welcher gegebenenfalls zusätzlich ein oder zwei Atome Stickstoff und/oder Sauerstoff als weitere Heteroatome im Ring enthält, sowie
Y¹ und Y² unabhängig voneinander jeweils eine Gruppe der Formel IVh
-NR⁹-D²-E¹ IVh
darstellen, in der R⁹ unabhängig von R⁸ ist und ein Substituent aus derselben Gruppe wie R⁸ bedeutet, und D² unabhängig von D¹ ist und dieselbe Gruppe von Substituenten wie D¹, CH₂ oder eine direkte Bindung bedeutet, und E¹ COOM oder SO₃M ist, worin M Wasserstoff, Alkalimetall oder die stöchiometrische Menge eines Erdalkalimetalls, oder ein Ammoniumion, welches gegebenenfalls durch aliphatische, aromatische und araliphatische Reste substituiert ist, und
Z eine Gruppe der Formel IVi
-NR¹⁰-Z*-NR¹⁰- IVi
darstellt, in der R¹⁰ Methyl oder Wasserstoff ist, und Z* die Gruppe C₂-C₆-Alkylen oder Phenylen ist, welches gegebenenfalls durch COOM oder SO₃M substituiert ist, wobei M die obengenannten Bedeutungen hat, oder wobei Z eine Gruppe der Formel IVj
-NH-C₂-C₃-Alkylen-Z**-C₂-C₃-Alkylen-NH- IVj
darstellt, in der Z** die Gruppe N-C₁-C₂₀-Alkyl, NH oder O bedeutet, und
m eine ganze Zahl von 1 bis 30 bedeutet.

4. Verfahren nach Anspruch 1, dadurch gekennzeichnet, daß die gesamte Menge an ZH₂ bereits vor dem Zusammengeben der beiden Reaktionsansätze im zweiten Reaktionsansatz vorhanden ist.

5. Verfahren zum Dispergieren, Emulgieren oder Verteilen von Feststoffen in wäßrigen Medien, dadurch gekennzeichnet, daß einer der in Anspruch 1, 2 oder 3 definierten Verbindungen der Formel IV verwendet wird.

6. Verfahren zum Dispergieren von Feststoffen, vorzugsweise von Mineralien, Pflanzenschutzmitteln und Farbstoffen, von Pigmenten und optischen Aufhellern, dadurch gekennzeichnet, daß eine der in Anspruch 1, 2 oder 3 definierten Verbindungen der Formel IV verwendet wird.

7. Pigmentdispersion, gekennzeichnet im wesentlichen durch den Gehalt an mindestens einer der Verbindungen der Formel IV gemäß Anspruch 1 und mindestens einem anorganischen und/oder organischen Pigment.

8. Verfahren zum Pigmentieren von wäßrigen oder wasserverdünnbaren Einbrennlacksystemen oder wäßrigen Bindemitteln, dadurch gekennzeichnet, daß eine Pigmentdispersion gemäß Anspruch 7 verwendet wird.

9. Pigmentzubereitung, gekennzeichnet im wesentlichen durch den Gehalt an
a) 99,5 bis 50 Gew.-% mindestens eines organischen Pigments,
b) 0,5 bis 30 Gew.-% mindestens einer Verbindung der Formel IV gemäß Anspruch 1 und
c) 0 bis 20 Gew.-% an weiteren üblichen Zusatzstoffen.

10. Pigmentzubereitung, gekennzeichnet im wesentlichen durch den Gehalt an
a) 97 bis 70 Gew.-% mindestens eines polycyclischen organischen Pigments,
b) 3 bis 20 Gew.-% mindestens einer Verbindung der Formel IV gemäß Anspruch 1 und
c) 0 bis 10 Gew.-% an weiteren üblichen Zusatzstoffen.

11. Verfahren zur Herstellung einer Pigmentzubereitung gemäß Anspruch 9 oder 10, dadurch gekennzeichnet, daß die Oberfläche der Pigmente mit mindestens einer Verbindung der Formel IV beschichtet wird.

12. Verwendung einer Pigmentzubereitung gemäß Anspruch 9 oder 10 zur Pigmentierung von wäßrigen oder wasserverdünnbaren Einbrennlacksystemen oder wäßrigen Bindemitteln.

## Claims (Claims for the following Contracting State(s): CH, DE, DK, FR, GB, IT, LI, NL)

1. A triazine compound of the formula IV 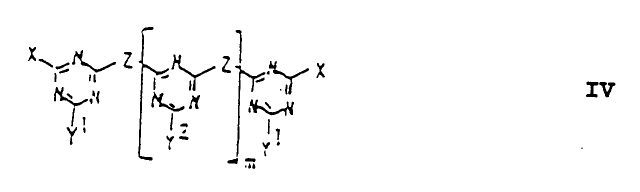 wherein
X is a group of the formula IVa
-A¹-D¹-NR¹R² IVa
in which A¹ is a bridge member NR³, wherein R³ is hydrogen or a C₁-C₂₂-alkyl radical or a C₃-C₂₂-alkenyl radical which is unsubstituted or substituted by an OH group,
D¹ is an arylene group or a branched or straight-chain C₂-C₁₂-alkylene group which may be interrupted by one or more bridge members selected from the group A², and A², independently of A¹, is the same group of substituents as A¹,
and R¹ and R², independently of one another, are branched or straight-chain C₁-C₂₀-alkyl groups or C₃-C₂₀-alkenyl groups, or in which R¹ and R², together with the nitrogen atom, form an unsaturated or saturated five- or six-membered ring which may additionally contain one or two nitrogen, oxygen or sulfur atoms as further hetero atoms in the ring,
or wherein X is a group of the formula IVb 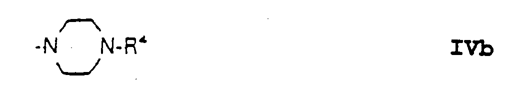 in which R⁴ is C₁-C₆-alkyl, and
Y¹ and Y², independently of one another, are each a group of the formula IVc
-A³-D²-E¹ IVc
in which A³ is independent of A¹ and is a bridge member selected from the same group of substituents as A¹,
D² is independent of D¹ and is the same group of substituents as D¹ or is CH₂ or a direct bond,
and E¹ is COOM, SO₃M, OSO₃M or OPO₃M₂, wherein M is hydrogen, a metal or the stoichiometric amount of an alkaline earth metal, or an ammonium ion which is unsubstituted or substituted by aliphatic, aromatic and araliphatic radicals, and
Z is a group of the formula IVd
-A⁴-Z*-A⁴- IVd
in which A⁴ is independent of A¹ and is a bridge member selected from the same group of substituents as A¹ and
Z* is the group CO, branched or straight-chain C₂-C₂₅-alkylene, C₅-C₆-cycloalkylene or C₆-C₁₄-arylene, which is unsubstituted or substituted by COOM, SO₃M, Cl, OH or alkoxy groups, in which M has the above-mentioned meanings, and wherein, in the cyclic compounds, some of the carbon atoms may be replaced by the hetero atoms nitrogen, oxygen or sulfur,
or wherein Z is a bridge member of the formulae IVe and IVf
-NH-C₆H₄-A⁵-C₆H₄-NH- IVe
-NH-C₂-C₃-alkylene-A⁵-C₂-C₃-alkylene-NH- IVf
in which A⁵ is a group of the formula CR⁵R⁶, NR⁷, O, SO, SO₂ or CO, in which R⁵ and R⁶, independently of one another, may be branched or straight-chain C₁-C₄-alkyl groups or hydrogen and wherein R⁷, independently of R³, is the same group of substituents as R³,
or Z is any combination of the groups of the formulae IVd, IVe and IVf, and
m is an integer from 1 to 100.

2. A triazine compound of the formula IV as claimed in claim 1, wherein
X is a group of the formula IVa
-A¹-D¹-NR¹R² IVa
in which
A¹ is a bridge member NR³, wherein R³ is hydrogen or a C₁-C₂₂-alkyl radical or a C₃-C₂₂-alkenyl radical which is unsubstituted or substituted by an OH group,
D¹ is an arylene group or a branched or straight-chain C₂-C₁₂-alkylene group which may be interrupted by one or more bridge members selected from the group A², and A², independently of A¹, is the same group of substituents as A¹, and
R¹ and R², independently of one another, are branched or straight-chain C₁-C₂₀-alkyl groups or C₃-C₂₀-alkenyl groups, or in which R¹ and R², together with the nitrogen atom, form an unsaturated or saturated five- or six-membered ring which may additionally contain one or two nitrogen or oxygen atoms as further hetero atoms in the ring, or wherein X is a group of the formula IVb 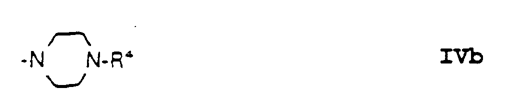 in which R⁴ is C₁-C₆-alkyl, and
Y¹ and Y², independently of one another, are each a group of the formula IVc
-A³-D²-E¹ IVc
in which A³ is independent of A¹ and is a bridge member selected from the same group of substituents as A¹, D² is independent of D¹ and is the same group of substituents as D¹ or is CH₂ or a direct bond, and E¹ is COOM or SO₃M, wherein M is hydrogen, an alkali metal or the stoichiometric amount of an alkaline earth metal, or an ammonium ion which is unsubstituted or substituted by aliphatic, aromatic and araliphatic radicals, and
Z is a group of the formula IVd
-A⁴-Z*-A⁴- IVd
in which A⁴ is independent of A¹ and is a bridge member selected from the same group of substituents as A¹ and Z* is the group CO, branched or straight-chain C₂-C₂₅-alkylene, C₅-C₆-cycloalkylene or phenylene, which is unsubstituted or substituted by COOM or SO₃M, wherein M has the above-mentioned meanings, and wherein, in the cyclic compounds, some of the carbon atoms may be replaced by the hetero atoms nitrogen or oxygen, or Z is a combination of the groups of the formula IVd, and
m is an integer from 1 to 100.

3. A triazine compound of the formula IV as claimed in claim 1, wherein
X is a group of the formula IVg
-NR⁸-D¹-NR¹R² IVg
in which R⁸ is methyl or hydrogen, and D¹ is a branched or straight-chain C₂-C₆-alkylene group or a phenylene group and R¹ and R², independently of one another, are branched or straight-chain C₁-C₆-alkyl groups, or in which R¹ and R², together with the nitrogen atom, form an unsaturated or saturated five- or six-membered ring which may additionally contain one or two nitrogen or oxygen atoms as further hetero atoms in the ring, and
Y¹ and Y², independently of one another, are each a group of the formula IVh
-NR⁹-D²-E¹ IVh
in which R⁹ is independent of R⁸ and is a substituent selected from the same group as R⁸, and D² is independent of D¹ and is the same group of substituents as D¹ or is CH₂ or a direct bond, and E¹ is COOM or SO₃M, wherein M is hydrogen, an alkali metal or the stoichiometric amount of an alkaline earth metal, or an ammonium ion which is unsubstituted or substituted by aliphatic, aromatic and araliphatic radicals, and
Z is a group of the formula IVi
-NR¹⁰-Z*-NR¹⁰- IVi
in which R¹⁰ is methyl or hydrogen and Z* is the group C₂-C₆-alkylene or phenylene, which is unsubstituted or substituted by COOM or SO₃M, wherein M has the abovementioned meanings, or wherein Z is a group of the formula IVj
-NH-C₂-C₃-alkylene-Z**-C₂-C₃-alkylene-NH- IVj
in which Z** is the group N-C₁-C₂₀-alkyl, NH or O, and
m is an integer from 1 to 30.

4. A process for the preparation of a compound as claimed in claim 1, wherein, in separate containers, first a compound of the formula Y¹-H (first reaction batch) and Y²-H (second reaction batch) are each reacted in aqueous solution with a cyanuric halide at a temperature of 0 to 10°C, then the intermediate formed in the first reaction batch is reacted with a compound of the formula X-H and the intermediate formed in the second reaction batch is reacted with a compound of the formula ZH₂, in each case at a temperature of from 20 to 60°C, and the two reaction batches are then combined and are reacted with 0.1 to 0.6 mol of ZH₂ per mol of cyanuric halide used at a temperature of 70 to 150°C.

5. The process as claimed in claim 4, wherein the total amount of ZH₂ is present in the second reaction batch even before the two reaction batches are combined.

6. Use of a compound as claimed in at least one of claims 1 to 3 as a surfactant.

7. Use of a compound as claimed in at least one of claims 1 to 3 as a dispersant, emulsifier or distributing agent for solids in aqueous media.

8. Use of a compound as claimed in at least one of claims 1 to 3 as a dispersant for solids, preferably for minerals, crop protection agents and dyes, for pigments and optical brighteners.

9. A pigment dispersion essentially containing at least one of the compounds of the formula IV as claimed in claim 1 and at least one inorganic or organic pigment.

10. Use of a pigment dispersion as claimed in claim 9 for pigmenting aqueous or water-dilutable baking enamel systems or aqueous binders.

11. A pigment preparation essentially containing
a) 99.5 to 50% by weight of at least one organic pigment,
b) 0.5 to 30% by weight of at least one compound of the formula IV as claimed in claim 1 and
c) 0 to 20% by weight of further conventional additives.

12. A pigment preparation essentially containing
a) 97 to 70% by weight of at Least one polycyclic organic pigment,
b) 3 to 20% by weight of at least one compound of the formula IV as claimed in claim 1 and
c) 0 to 10% by weight of further conventional additives.

13. A process for the preparation of a pigment preparation as claimed in claim 11 or 12, wherein the surface of the pigments is coated with at least one compound of the formula IV.

14. Use of a pigment preparation as claimed in claim 11 or 12 for pigmenting aqueous or water-dilutable baking enamel systems or aqueous binders.

## Claims (Claims for the following Contracting State(s): ES)

1. A process for the preparation of a triazine compound of the formula IV 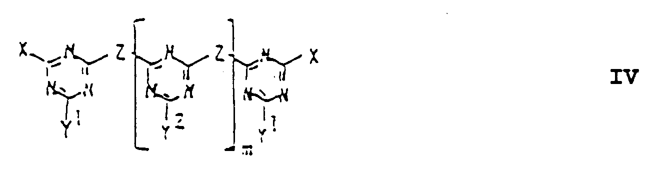 wherein
X is a group of the formula IVa
-A¹-D¹-NR¹R² IVa
in which A¹ is a bridge member NR³, wherein R³ is hydrogen or a C₁-C₂₂-alkyl radical or a C₃-C₂₂-alkenyl radical which is unsubstituted or substituted by an OH group,
D¹ is an arylene group or a branched or straight-chain C₂-C₁₂-alkylene group which may be interrupted by one or more bridge members selected from the group A², and A², independently of A¹, is the same group of substituents as A¹, and R¹ and
R², independently of one another, are branched or straight-chain C₁-C₂₀-alkyl groups or C₃-C₂₀-alkenyl groups, or in which R¹ and R², together with the nitrogen atom, form an unsaturated or saturated five- or six-membered ring which may additionally contain one or two nitrogen, oxygen or sulfur atoms as further hetero atoms in the ring,
or wherein X is a group of the formula IVb 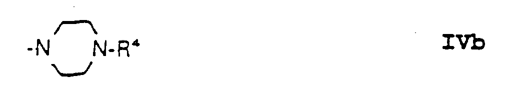 in which R⁴ is C₁-C₆-alkyl, and
Y¹ and Y², independently of one another, are each a group of the formula IVc
-A³-D²-E¹ IVc
in which A³ is independent of A¹ and is a bridge member selected from the same group of substituents as A¹,
D² is independent of D¹ and is the same group of substituents as D¹ or is CH₂ or a direct bond,
and E¹ is COOM, SO₃M, OSO₃M or OPO₃M₂, wherein M is hydrogen, a metal or the stoichiometric amount of an alkaline earth metal, or an ammonium ion which is unsubstituted or substituted by aliphatic, aromatic and araliphatic radicals, and
Z is a group of the formula IVd
-A⁴-Z*-A⁴- IVd
in which A⁴ is independent of A¹ and is a bridge member selected from the same group of substituents as A¹ and
Z* is the group CO, branched or straight-chain C₂-C₂₅-alkylene, C₅-C₆-cycloalkylene or C₆-C₁₄-arylene, which is unsubstituted or substituted by COOM, SO₃M, Cl, OH or alkoxy groups, wherein M has the above-mentioned meanings, and wherein, in the cyclic compounds, some of the carbon atoms may be replaced by the hetero atoms nitrogen, oxygen or sulfur,
or wherein Z is a bridge member of the formulae IVe and IVf
-NH-C₆H₄-A⁵-C₆H₄-NH- IVe
-NH-C₂-C₃-alkylene-A⁵-C₂-C₃-alkylene-NH- IVf
in which A⁵ is a group of the formula CR⁵R⁶, NR⁷, O, SO, SO₂ or CO, in which R⁵ and R⁶, independently of one another, may be branched or straight-chain C₁-C₄-alkyl groups or hydrogen and wherein R⁷, independently of R³, is the same group of substituents as R³,
or Z is any combination of the groups of the formulae IVd, IVe and IVf, and
m is an integer from 1 to 100,
wherein, in separate containers, first a compound of the formula Y¹-H (first reaction batch) and Y²-H (second reaction batch) are each reacted in aqueous solution with a cyanuric halide at a temperature of 0 to 10°C, then the intermediate formed in the first reaction batch is reacted with a compound of the formula X-H and the intermediate formed in the second reaction batch is reacted with a compound of the formula ZH₂, in each case at a temperature of 20 to 60°C, and the two reaction batches are then combined and are reacted with 0.1 to 0.6 mol of ZH₂ per mol of cyanuric halide used at a temperature of 70 to 150°C.

2. The process as claimed in claim 1, wherein
X is a group of the formula IVa
-A¹-D¹-NR¹R² IVa
in which
A¹ is a bridge member NR³, wherein R³ is hydrogen or a C₁-C₂₂-alkyl radical or a C₃-C₂₂-alkenyl radical which is unsubstituted or substituted by an OH group,
D¹ is an arylene group or a branched or straight-chain C₂-C₁₂-alkylene group which may be interrupted by one or more bridge members selected from the group A², and A², independently of A¹, is the same group of substituents as A¹, and
R¹ and R², independently of one another, are branched or straight-chain C₁-C₂₀-alkyl groups or C₃-C₂₀-alkenyl groups, or in which R¹ and R², together with the nitrogen atom, form an unsaturated or saturated five- or six-membered ring which may additionally contain one or two nitrogen or oxygen atoms as further hetero atoms in the ring, or wherein X is a group of the formula IVb 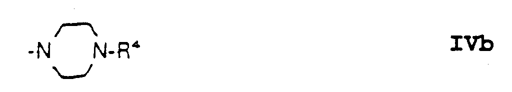 in which R⁴ is C₁-C₆-alkyl, and
Y¹ and Y², independently of one another, are each a group of the formula IVc
-A³-D²-E¹ IVc
in which A³ is independent of A¹ and is a bridge member selected from the same group of substituents as A¹, D² is independent of D¹ and is the same group of substituents as D¹ or is CH₂ or a direct bond, and E¹ is COOM or SO₃M, wherein M is hydrogen, an alkali metal or the stoichiometric amount of an alkaline earth metal, or an ammonium ion which is unsubstituted or substituted by aliphatic, aromatic and araliphatic radicals, and
Z is a group of the formula IVd
-A⁴-Z*-A⁴- IVd
in which A⁴ is independent of A¹ and is a bridge member selected from the same group of substituents as A¹ and Z* is the group CO, branched or straight-chain C₂-C₂₅-alkylene, C₅-C₆-cycloalkylene or phenylene, which is unsubstituted or substituted by COOM or SO₃M, wherein M has the above-mentioned meanings, and wherein, in the cyclic compounds, some of the carbon atoms may be replaced by the hetero atoms nitrogen or oxygen, or Z is a combination of the groups of the formula IVd, and
m is an integer from 1 to 100.

3. The process as claimed in claim 1, wherein
X is a group of the formula IVg
-NR⁸-D¹-NR¹R² IVg
in which R⁸ is methyl or hydrogen, and D¹ is a branched or straight-chain C₂-C₆-alkylene group or a phenylene group and R¹ and R², independently of one another, are branched or straight-chain C₁-C₆-alkyl groups, or in which R¹ and R², together with the nitrogen atom, form an unsaturated or saturated five- or six-membered ring which may additionally contain one or two nitrogen or oxygen atoms as further hetero atoms in the ring, and
Y¹ and Y², independently of one another, are each a group of the formula IVh
-NR⁹-D²-E¹ IVh
in which R⁹ is independent of R⁸ and is a substituent selected from the same group as R⁸, and D² is independent of D¹ and is the same group of substituents as D¹ or is CH₂ or a direct bond, and E¹ is COOM or SO₃M, wherein M is hydrogen, an alkali metal or the stoichiometric amount of an alkaline earth metal, or an ammonium ion which is unsubstituted or substituted by aliphatic, aromatic and araliphatic radicals, and
Z is a group of the formula IVi
-NR¹⁰-Z*-NR¹⁰- IVi
in which R¹⁰ is methyl or hydrogen and Z* is the group C₂-C₆-alkylene or phenylene, which is unsubstituted or substituted by COOM or SO₃M, wherein M has the abovementioned meanings, or wherein Z is a group of the formula IVj
-NH-C₂-C₃-alkylene-Z**-C₂-C₃-alkylene-NH- IVj
in which Z** is the group N-C₁-C₂₀-alkyl, NH or O, and
m is an integer from 1 to 30.

4. The process as claimed in claim 1, wherein the total amount of ZH₂ is present in the second reaction batch even before the two reaction batches are combined.

5. A process for dispersing, emulsifying or distributing solids in aqueous media, which comprises using one of the compounds of the formula IV defined in claim 1, 2 or 3.

6. A process for dispersing solids, preferably minerals, crop protection agents and dyes, pigments and optical brighteners, which comprises using one of the compounds of the formula IV defined in claim 1, 2 or 3.

7. A pigment dispersion essentially containing at least one of the compounds of the formula IV as claimed in claim 1 and at least one inorganic or organic pigment.

8. A process for pigmenting aqueous or water-dilutable baking enamel systems or aqueous binders, which comprises using a pigment dispersion as claimed in claim 7.

9. A pigment preparation essentially containing
a) 99.5 to 50% by weight of at least one organic pigment,
b) 0.5 to 30% by weight of at least one compound of the formula IV as claimed in claim 1 and
c) 0 to 20% by weight of further conventional additives.

10. A pigment preparation essentially containing
a) 97 to 70% by weight of at least one polycyclic organic pigment,
b) 3 to 20% by weight of at least one compound of the formula IV as claimed in claim 1 and
c) 0 to 10% by weight of further conventional additives.

11. A process for the preparation of a pigment preparation as claimed in claim 9 or 10, wherein the surface of the pigments is coated with at least one compound of the formula IV.

12. Use of a pigment preparation as claimed in claim 9 or 10 for pigmenting aqueous or water-dilutable baking enamel systems or aqueous binders.

## Revendications (Revendications pour l'(les) Etat(s) contractant(s) suivant(s): CH, DE, DK, FR, GB, IT, LI, NL)

1. Composés de triazine de formule générale IV 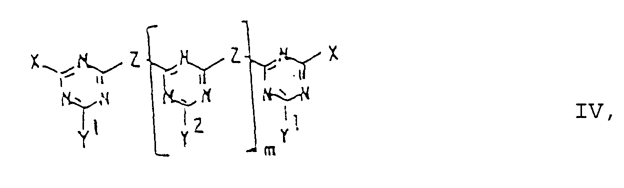
X étant un groupe de formule IVa
-A¹-D¹-NR¹R² IVa
dans laquelle A¹ représente un pont NR³, dans lequel R³ représente l'hydrogène ou un radical alkyle en C₁-C₂₂ ou un radical alcényle en C₃-C₂₂ qui peut être éventuellement substitué par un groupe OH,
D¹ représente un groupe arylène ou un groupe alkylène en C₂-C₁₂ linéaire ou ramifié qui peut être éventuellement interrompu par un ou plusieurs ponts du groupe A², et A², indépendamment de A¹, représente le même groupe de substituants que A¹, et
R¹ et R², indépendamment l'un de l'autre, représentent des groupes alkyle en C₁-C₂₀ ou des groupes alcényle en C₃-C₂₀ linéaires ou ramifiés, ou dans laquelle R¹ et R² ensemble avec l'atome d'azote forment un cycle insaturé ou saturé à 5 ou 6 chaînons, lequel peut être éventuellement substitué en plus par 1 ou 2 atomes d'azote, d'oxygène et/ou de soufre en tant que d'autres hétéroatomes dans le cycle, ou
X représentant un groupe de formule IVb 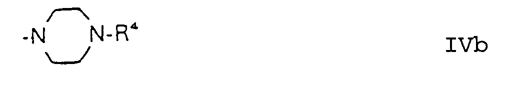 dans laquelle R⁴ représente alkyle en C₁-C₆,
Y¹ et Y², indépendamment l'un de l'autre, représentent chacun un groupe de formule IVc
-A³-D²-A¹ IVc
dans laquelle A³ est indépendant de A¹ et représente un élément de pont du même groupe des substituants que A¹,
D² est indépendant de D¹ et représente le même groupe de substituants que D¹, CH₂ ou une liaison directe, et
E¹ représente COOM, SO₃M, OSO₃M ou OPO₃M₂, où M représente l'hydrogène, un métal ou la quantité stoechiométrique d'un métal alcalino-terreux ou un ion ammonium, lequel est éventuellement substitué par des radicaux aliphatiques, aromatiques et araliphatiques, et
Z représente un groupe de formule IVd
-A⁴-Z*-A⁴ IVd
dans laquelle A⁴ est indépendant de A¹ et représente un élément de pont du même groupe de substituants que A¹, et
Z* représente le groupe CO, alkylène en C₂-C₂₅ linéaire ou ramifié, cycloalkylène en C₅-C₆ ou arylène en C₆-C₁₄, lequel est éventuellement substitué par COOM, SO₃M, Cl, OH ou groupes alcoxy, M ayant les significations données ci-dessus, et dans les composés cycliques les atomes de carbone sont éventuellement substitués en partie par des hétéroatomes l'azote, l'oxygène et/ou le soufre, ou
Z étant un pont de formules générales IVe et IVf
-NH-C₆H₄-A⁵-C₆H₄-NH- IVe
-NH-(alkylène en C₂-C₃)-A⁵-(alkylène en C₂-C₃)NH- IVf
dans lesquelles A⁵ représente un groupe de formules CR⁵R⁶, NR⁷, O, SO, SO₂ et CO, R⁵ et R⁶, indépendamment l'un de l'autre, pouvant être des groupes alkyle en C₁-C₄ linéaires ou ramifiés ainsi que l'hydrogène et R⁷, indépendamment de R³, représentant le même groupe de substituants que R³, ou
Z signifiant une combinaison quelconque des groupes de formules IVd, IVe et/ou IVf, et
m est un nombre entier de 1 à 100.

2. Composé de triazine de formule générale IV selon la revendication 1 dans laquelle
X étant un groupe de formule IVa
-A¹-D¹-NR¹R² IVa
dans laquelle A¹ représente un pont NR³, dans lequel R³ représente l'hydrogène ou un radical alkyle en C₁-C₂₂ ou un radical alcényle en C₃-C₂₂ qui peut être éventuellement substitué par un groupe OH,
D¹ représente un groupe arylène ou un groupe alkylène en C₂-C₁₂ linéaire ou ramifié, qui peut être éventuellement interrompu par un ou plusieurs ponts du groupe A², et A², indépendamment de A¹, représente le même groupe de substituants que A¹, et
R¹ et R², indépendamment l'un de l'autre, représentent des groupes alkyle en C₁-C₂₀ ou des groupes alcényle en C₃-C₂₀ linéaires ou ramifiés, ou dans laquelle R¹ et R² ensemble avec l'atome d'azote forment un cycle insaturé ou saturé à 5 ou 6 chaînons, lequel peut être éventuellement substitué en plus par 1 ou 2 atomes d'azote, d'oxygène et/ou de soufre en tant que d'autres hétéroatomes dans le cycle, ou
X représentant un groupe de formule IVb 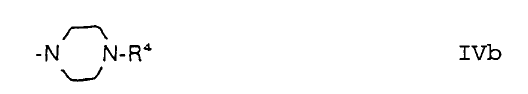 dans laquelle R⁴ représente alkyle en C₁-C₆, ainsi que
Y¹ et Y², indépendamment l'un de l'autre, représentent chacun un groupe de formule IVc
-A³-D²-E¹ IVc
dans laquelle A³ est indépendant de A¹ et représente un pont du même groupe des substituants que A¹, D² est indépendant de D¹ et représente le même groupe de substituants que D¹, CH₂ ou une liaison directe, et E¹ représente COOM ou SO₃M, où M représente l'hydrogène, un métal alcalin ou la quantité stoechiométrique d'un métal alcalino-terreux ou un ion ammonium lequel est éventuellement substitué par des radicaux aliphatiques, aromatiques et araliphatiques, et
Z représente un groupe de formule IVd
-A⁴-Z*-A⁴ IVd
dans laquelle A⁴ est indépendant de A¹ et représente un pont du même groupe de substituants que A¹, et Z* représente le groupe CO, alkylène en C₂-C₂₅ linéaire ou ramifié, cycloalkylène en C₅-C₆ ou phénylène, lequel est éventuellement substitué par COOM ou SO₃M, M ayant les significations données ci-dessus, et dans les composés cycliques les atomes de carbone étant éventuellement substitués par des hétéroatomes l'azote, et/ou l'oxygène, ou Z représente une combinaison des groupes de formule IVd, et
m est un nombre entier de 1 à 100.

3. Composé de triazine de formule générale IV selon la revendication 1 dans laquelle
X étant un groupe de formule IVg
-NR⁸-D¹-NR¹R² IVg
dans laquelle R⁸ représente méthyle ou l'hydrogène, et D¹ étant un groupe alkylène en C₂-C₅ linéaire ou ramifié ou un groupe phénylène, et R¹ et R², indépendamment l'un de l'autre, représentent des groupes alkyle en C₁-C₆ linéaire ou ramifié, ou dans laquelle R¹ et R² ensemble avec l'atome d'azote forment un cycle insaturé ou saturé à 5 ou 6 chaînons, lequel peut contenir encore éventuellement 1 ou 2 atomes d'azote et/ou d'oxygène en tant que d'autres hétéroatomes dans le cycle, ainsi que
Y¹ et Y², indépendamment l'un de l'autre, représentent chacun un groupe de formule IVh
-NR⁹-D²-E¹ IVh
dans laquelle R⁹ est indépendant de R⁸ et représente un substituant du même groupe que R⁸, et D² est indépendant de D¹ et représente le même groupe de substituants que D¹, CH₂ ou une liaison directe, et E¹ représente COOM ou SO₃M, où M représente l'hydrogène, un métal alcalin ou la quantité stoechiométrique d'un métal alcalino-terreux, ou un ion ammonium lequel est éventuellement substitué par des radicaux aliphatiques, aromatiques et araliphatiques, et
Z représente un groupe de formule IVi
-NR¹⁰-Z*-NR¹⁰- IVi
dans laquelle R¹⁰ représente méthyle ou l'hydrogène, et Z* représente le groupe alkylène en C₂-C₅ ou phénylène, lequel est éventuellement substitué par COOM ou SO₃M, M ayant les significations données ci-dessus, ou Z étant un groupe de formule IVj
-NH-(alkylène en C₂-C₃)-Z**-(alkylène en C₂-C₃)-NH- IVj
dans laquelle Z** représente le groupe N-(alkyle en C₁-C₂₀), NH ou O, et
m est un nombre entier de 1 à 30.

4. Procédé pour la préparation d'un composé selon la revendication 1, caractérisé en ce que l'on fait réagir dans des récipients séparés d'abord un composé de formule Y¹-H (première charge réactionnelle) et Y²-H (deuxième charge réactionnelle), chacun en solution aqueuse, avec l'halogénure de cyanuryle à une température de 0 à 10 °C, ensuite, on fait réagir le produit intermédiaire formé dans la première charge réactionnelle avec un composé de formule X-H et le produit intermédiaire formé dans la deuxième charge réactionnelle avec un composé de formule ZH₂, toujours à une température de 20 à 60 °C, ensuite, on réunit les deux charges réactionnelles et on fait réagir avec 0,1 à 0,6 mode de ZH₂ par mode d'halogénure de cyanuryle utilisée à une température de 70 à 150 °C.

5. Procédé selon la revendication 4, caractérisé en ce que la quantité totale en ZH₂ est déjà présente dans la deuxième charge réactionnelle avant la réunion des deux charges réactionnelles.

6. Utilisation des composés selon au moins l'une des revendications 1 à 3, en tant qu'agent tensio-actif.

7. Utilisation des composés selon au moins l'une des revendications 1 à 3, en tant qu'agents dispersants, émulsionnants ou de distribution des matières solides en milieu aqueux.

8. Utilisation des composés selon au moins l'une des revendications 1 à 3, en tant qu'agents dispersants de matières solides, de préférence de minerais, d'agents phytoprotecteurs et de colorants, de pigment et d'azurants optiques.

9. Dispersion pigmentaire caractérisée essentiellement par la teneur en au moins un composé de formule IV selon la revendication 1, et au moins un pigment minéral et/ou organique.

10. Utilisation de la dispersion pigmentaire selon la revendication 9, pour la pigmentation de systèmes aqueux de vernis à cuire ou diluables par l'eau ou de liants aqueux.

11. Préparation pigmentaire caractérisée essentiellement par la teneur en
a) de 99,5 à 50 % en poids d'au moins un pigment organique,
b) de 0,5 à 30 % en poids d'au moins un composé de formule IV selon la revendication 1, et
c) de 0 à 20 % en poids en d'autres additifs usuels.

12. Préparation pigmentaire caractérisée essentiellement par sa teneur en
a) de 97 à 70 % en poids d'un pigment organique polycyclique,
b) de 3 à 20 % en poids d'au moins un composé de formule IV selon la revendication 1 et
c) de 0 à 10 % en poids en d'autres additifs usuels.

13. Procédé pour la préparation d'une préparation pigmentaire selon les revendications 11 et 12, caractérisé en ce que la surface des pigments est revêtue d'au moins un composé de formule IV.

14. Utilisation d'une préparation pigmentaire selon la revendication 11 ou 12 pour la pigmentation de systèmes de vernis à cuire aqueux ou diluables par l'eau ou d'agents liants aqueux.

## Revendications (Revendications pour l'(les) Etat(s) contractant(s) suivant(s): ES)

1. Procédé pour la préparation d'un composé de triazine de formule générale IV 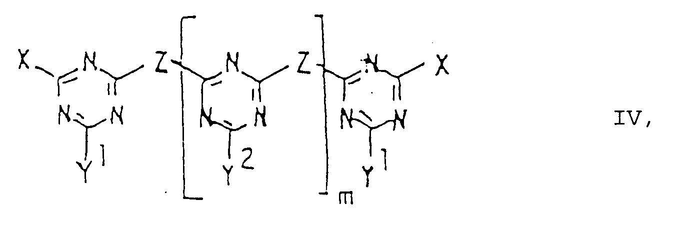
X étant un groupe de formule IVa
-A¹-D¹-NR¹R² IVa
dans laquelle A¹ représente un pont NR³, dans lequel R³ représente l'hydrogène ou un radical alkyle en C₁-C₂₂ ou un radical alcényle en C₃-C₂₂ qui peut être éventuellement substitué par un groupe OH,
D¹ représente un groupe arylène ou un groupe alkylène en C₂-C₁₂ linéaire ou ramifié qui peut être éventuellement interrompu par un ou plusieurs ponts du groupe A², et A², indépendamment de A¹, représente le même groupe de substituants que A¹, et
R¹ et R², indépendamment l'un de l'autre, représentent des groupes alkyle en C₁-C₂₀ ou des groupes alcényle en C₃-C₂₀ linéaires ou ramifiés, ou dans laquelle R¹ et R² ensemble avec l'atome d'azote forment un cycle insaturé ou saturé à 5 ou 6 chaînons, lequel peut être éventuellement substitué en plus par 1 ou 2 atomes d'azote, d'oxygène et/ou de soufre en tant que d'autres hétéroatomes dans le cycle, ou
X représentant un groupe de formule IVb 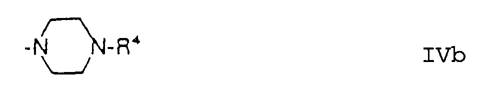 dans laquelle R⁴ représente alkyle en C₁-C₆, ainsi que
Y¹ et Y², indépendamment l'un de l'autre, représentent chacun un groupe de formule IVc
-A³-D²-A¹ IVc
dans laquelle A³ est indépendant de A¹ et représente un pont du même groupe des substituants que A¹,
D² est indépendant de D¹ et représente le même groupe de substituants que D¹, CH₂ ou une liaison directe, et
E¹ représente COOM, SO₃M, OSO₃M ou OPO₃M₂, où M représente l'hydrogène, un métal ou la quantité stoechiométrique d'un métal alcalino-terreux ou un ion ammonium, lequel est éventuellement substitué par des radicaux aliphatiques, aromatiques et araliphatiques, et
Z représente un groupe de formule IVd
-A⁴-Z*-A⁴ IVd
dans laquelle A⁴ est indépendant de A¹ et représente un pont du même groupe de substituants que A¹, et
Z* représente le groupe CO, alkylène en C₂-C₂₅ linéaire ou ramifié, cycloalkylène en C₅-C₆ ou arylène en C₆-C₁₄, lequel est éventuellement substitué par COOM, SO₃M, Cl, OH ou groupes alcoxy, M ayant les significations données ci-dessus, et dans les composés cycliques les atomes de carbone sont éventuellement substitués en partie par des hétéroatomes l'azote, l'oxygène et/ou le soufre, ou
Z étant un pont de formules générales IVe et IVf
-NH-C₆H₄-A⁵-C₆H₄-NH- IVe
-NH-(alkylène en C₂-C₃)-A⁵-(alkylène en C₂-C₃)-NH- IVf
dans lesquelles A⁵ représente un groupe de formules CR⁵R⁶, NR⁷, O, SO, SO₂ et CO, R⁵ et R⁶, indépendamment l'un de l'autre, pouvant être des groupes alkyle en C₁-C₄ linéaire ou ramifié ainsi que l'hydrogène et R⁷, indépendamment de R³, représentant le même groupe de substituants que R³, ou
Z signifiant une combinaison quelconque des groupes de formules IVd, IVe et/ou IVf, et
m est un nombre entier de 1 à 100,
caractérisé en ce que l'on fait réagir dans des récipients séparés d'abord un composé de formule Y¹-H (première charge réactionnelle) et Y²-H (deuxième charge réactionnelle), chacun en solution aqueuse, avec l'halogénure de cyanuryle à une température de 0 à 10 °C, ensuite, on fait réagir le produit intermédiaire formé dans la première charge réactionnelle avec un composé de formule X-H et le produit intermédiaire formé dans la deuxième charge réactionnelle avec un composé de formule ZH₂, toujours à une température de 20 à 60 °C, ensuite, on réunit les deux charges réactionnelles et on fait réagir avec 0,1 à 0,6 mole de ZH₂ par mole d'halogénure de cyanuryle utilisée à une température de 70 à 150 °C.

2. Procédé selon la revendication 1, caractérisé en ce que
X étant un groupe de formule IVa
-A¹-D¹-NR¹R² IVa
dans laquelle A¹ représente un pont NR³, dans lequel R³ représente l'hydrogène ou un radical alkyle en C₁-C₂₂ ou un radical alcényle en C₃-C₂₂ qui peut être éventuellement substitué par un groupe OH,
D¹ représente un groupe arylène ou un groupe alkylène en C₂-C₁₂ linéaire ou ramifié qui peut être éventuellement interrompu par un ou plusieurs ponts du groupe A², et A², indépendamment de A¹, représente le même groupe de substituants que A¹, et
R¹ et R², indépendamment l'un de l'autre, représentent des groupes alkyle en C₁-C₂₀ ou des groupes alcényle en C₃-C₂₀ linéaire ou ramifié, ou dans laquelle R¹ et R² ensemble avec l'atome d'azote forment un cycle insaturé ou saturé à 5 ou 6 chaînons, lequel peut être éventuellement substitué en plus par 1 ou 2 atomes d'azote, d'oxygène et/ou de soufre en tant que d'autres hétéroatomes dans le cycle, ou
X représentant un groupe de formule IVb 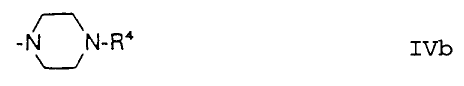 dans laquelle R⁴ représente alkyle en C₁-C₆,
Y¹ et Y², indépendamment l'un de l'autre, représentent chacun un groupe de formule IVc
-A³-D²-E¹ IVc
dans laquelle A³ est indépendant de A¹ et représente un pont du même groupe des substituants que A¹, D² est indépendant de D¹ et représente le même groupe de substituants que D¹, CH₂ ou une liaison directe, et E¹ représente COOM ou SO₃M, où M représente l'hydrogène, un métal alcalin ou la quantité stoechiométrique d'un métal alcalino-terreux ou un ion ammonium lequel est éventuellement substitué par des radicaux aliphatiques, aromatiques et araliphatiques, et
Z représente un groupe de formule IVd
-A⁴-Z*-A⁴ IVd
dans lequel A⁴ est indépendant de A¹ et représente un élément de pont du même groupe de substituants que A¹, et Z* représente le groupe CO, alkylène en C₂-C₂₅ linéaire ou ramifié, cycloalkylène en C₅-C₆ ou phénylène, lequel est éventuellement substitué par COOM ou SO₃M, M ayant les significations données ci-dessus, et dans les composés cycliques les atomes de carbone étant éventuellement substitués par des hétéroatomes l'azote, et/ou l'oxygène, ou Z représente une combinaison des groupes de formule IVd, et
m est un nombre entier de 1 à 100.

3. Procédé selon la revendication 1 caractérisé en ce que
X étant un groupe de formule IVg
-NR⁸-D¹-NR¹R² IVg
dans laquelle R⁸ représente méthyle ou l'hydrogène, et D¹ étant un groupe alkylène en C₂-C₅ linéaire ou ramifié ou un groupe phénylène, et R¹ et R², indépendamment l'un de l'autre, représentent des groupes alkyle en C₁-C₆ linéaire ou ramifié, ou dans laquelle R¹ et R² ensemble avec l'atome d'azote forment un cycle insaturé ou saturé à 5 ou 6 chaînons, lequel peut contenir encore éventuellement 1 ou 2 atomes d'azote et/ou d'oxygène en tant qu'autres hétéroatomes dans le cycle,
Y¹ et Y², indépendamment l'un de l'autre, représentent chacun un groupe de formule IVh
-NR⁹-D²-E¹ IVh
dans laquelle R⁹ est indépendant de R⁸ et représente un substituant du même groupe que R⁸, et D² est indépendant de D¹ et représente le même groupe de substituants que D¹, CH₂ ou une liaison directe, et E¹ représente COOM ou SO₃M, où M représente l'hydrogène, un métal alcalin ou la quantité stoechiométrique d'un métal alcalino-terreux, ou un ion ammonium, lequel est éventuellement substitué par des radicaux aliphatiques, aromatiques et araliphatiques, et
Z représente un groupe de formule IVi
-NR¹⁰-Z*-NR¹⁰- IVi
dans laquelle R¹⁰ représente méthyle ou l'hydrogène, et Z* représente le groupe alkylène en C₂-C₅ ou phénylène, lequel est éventuellement substitué par COOM ou SO₃M, M ayant les significations données ci-dessus, ou Z étant un groupe de formule IVj
-NH-(alkylène en C₂-C₃)-Z**-(alkylène en C₂-C₃)-NH- IVj
dans lequel Z** représente le groupe N-(alkyle en C₁-C₂₀), NH ou O, et
m est un nombre entier de 1 à 30.

4. Procédé selon la revendication 1, caractérisé en ce que la quantité totale en ZH₂ est déjà présente dans la deuxième charge réactionnelle avant la réunion des deux charges réactionnelles.

5. Procédé pour la dispersion, l'émulsionnement ou la distribution de matières solides en milieu aqueux, caractérisé en ce que l'on utilise un composé de formule IV défini selon les revendications 1, 2 ou 3.

6. Procédé pour la dispersion de matières solides, de préférence de minerais, d'agents phytoprotecteurs ou de colorants, de pigment et d'azurants optiques, caractérisé en ce que l'on utilise un composé de formule IV défini dans les revendications 1, 2 ou 3.

7. Dispersion pigmentaire caractérisée essentiellement par la teneur en au moins un composé de formule IV de la revendication 1 et au moins un pigment minéral et/ou organique.

8. Procédé pour la pigmentation de systèmes aqueux de vernis à cuire ou diluables par l'eau, caractérisé en ce que l'on utilise une dispersion pigmentaire selon la revendication 7.

9. Préparation pigmentaire caractérisée essentiellement par la teneur en
a) de 99,5 à 50 % en poids d'au moins un pigment organique,
b) de 0,5 à 30 % en poids d'au moins un composé de formule IV selon la revendication 1 et
c) de 0 à 20 % en poids en d'autres additifs usuels.

10. Préparation pigmentaire caractérisée essentiellement par la teneur en
a) de 97 à 70 % en poids d'un pigment organique polycyclique,
b) de 3 à 20 % en poids d'au moins un composé de formule IV selon la revendication 1 et
c) de 0 à 10 % en poids en d'autres additifs usuels.

11. Procédé pour la préparation d'une préparation pigmentaire selon la revendication 9 ou 10, caractérisé en ce que la surface des pigments est revêtue d'au moins un composé de formule IV.

12. Utilisation d'une préparation pigmentaire selon la revendication 9 ou 10, pour la pigmentation de systèmes de vernis à cuire aqueux ou diluables par l'eau ou d'agents liants aqueux.
